# EUROPEAN PATENT APPLICATION

(11) **EP 4 745 241 A1**
(43) Date of publication of application: **20.05.2026**
(21) Application number: 24838818.3
(22) Date of filing: 10.07.2024
(51) Int. Cl.: C12N 15/13, C12N 15/864, C12N 15/10, A61K 39/395, A61P 37/08, A61P 11/06, A61P 17/00

(54) **GENE THERAPY VECTOR NUCLEIC ACID CONSTRUCT FOR ALLERGIC DISEASES, AND USAGE METHOD THEREOF**

(30) Priority: 11.07.2023 CN 202310849120
(71) Applicant: KANGLIN BIOTECHNOLOGY (HANGZHOU) CO., LTD., Hangzhou, Zhejiang 310018 (CN)
(72) Inventor: WU, Haoquan, Hangzhou, Zhejiang 310018 (CN); SU, Lingling, Hangzhou, Zhejiang 310018 (CN); DING, Yanfu, Hangzhou, Zhejiang 310018 (CN)
(74) Representative: Bayramoglu et al.
(86) International application number: PCT/CN2024/104607
(87) International publication number: WO 2025/011566

(57) **Abstract**

A gene therapy vector nucleic acid construct for allergic diseases and use thereof are provided. Specifically, an expression cassette is provided. The expression cassette as described herein comprises the following elements: a promoter, a nucleotide sequence encoding an optional first signal peptide, a nucleotide sequence encoding a heavy chain of an anti-IgE antibody, a nucleotide sequence encoding an optional linker, a nucleotide sequence encoding an optional second signal peptide, and a nucleotide sequence encoding a light chain of an anti-IgE antibody. Further an adeno-associated virus vector comprising the expression cassette is provided. A gene expression framework of the adeno-associated virus vector enables high-level expression of a target gene, and the adeno-associated virus vector is useful for treating conditions, such as allergic asthma and chronic spontaneous urticaria.

## Description

The present application is based upon and claims priority to Chinese Patent Application No. 202310849120.8, filed on July 11, 2023, the entire contents of which are incorporated herein by reference.

### TECHNICAL FIELD

The present disclosure relates to the field of biomedical technology, and specifically relates to a gene therapy vector nucleic acid construct for allergic diseases, and methods of use thereof. A gene sequence construct may be used for the gene therapy of allergic diseases, such as chronic spontaneous urticaria and allergic asthma. The gene sequence construct enables more efficient and cost-effective production of adeno-associated virus vectors for the gene therapy of allergic diseases.

### BACKGROUND TECHNOLOGY

Allergic asthma is a chronic respiratory disease characterized by reversible airflow obstruction, which is associated with excessive mucus production and airway remodeling. It is commonly found in children, with a reported prevalence rate ranging from 5% to 15%. Over the past few decades, the prevalence and mortality rates of asthma have risen worldwide. It is estimated that asthma causes approximately 250,000 deaths each year and affects about 300 million people globally. The disease is caused by immune dysfunction, primarily influenced by effector T cell subsets, and is characterized by chronic airway inflammation involving multiple cell types, such as eosinophils, mast cells, T lymphocytes, neutrophils, and airway epithelial cells. This chronic inflammation is associated with airway remodeling and the development of airway hyperresponsiveness. This condition usually manifests as widespread, variable and reversible expiratory airflow limitation, which leads to recurrent symptoms, such as wheezing, shortness of breath, chest tightness, and/or cough. These symptoms vary in intensity over time. Overall, allergic asthma is a heterogeneous disease with multiple phenotypes. In addition to inflammation caused by increased airway eosinophils, increased Th2 cells, and excessive secretion of type 2 cytokines, such as IL-4, IL-5, and IL-13, patients with allergic asthma also exhibit a phenotype characterized by elevated serum allergen-specific IgE levels. Although most patients respond well to safe and evidence-based stepwise drug therapy, fewer than 5% develop a severe, treatment-resistant asthma phenotype.

Studies have shown that the autoimmune mechanism in most patients with allergic asthma involves the high-affinity receptor FcεRI for the Fc region of IgE. IgE is the primary immunoglobulin mediating type I allergic reactions. IgE binding to the high-affinity receptor FcεRI on the surface of mast cells or basophils induces degranulation, which results in the release of bioactive mediators, such as histamine, proteases, and cytokines, and producing platelet-activating factor and other arachidonic acid metabolites, such as prostaglandin D2, leukotrienes C4, D4, and E4. These mediators may induce airway smooth muscle contraction, capillary dilation with increased permeability, and increased glandular secretions. Collectively, they trigger both early and late allergic reactions that severely impact the quality of life of asthma patients.

Elevated serum specific IgE levels are a major hallmark of allergic asthma and a key pathogenic driver in the development of asthma and other allergic diseases. Therefore, how to reduce specific IgE levels in asthma patients has become a research focus for asthma prevention and treatment, and anti-IgE therapy is expected to provide a novel and effective therapeutic approach for asthma patients. In 2003, Omalizumab, the first humanized monoclonal antibody for anti-IgE therapy, was approved in the United States for the treatment of moderate to severe persistent allergic asthma. Omalizumab is capable of binding to the constant region (Cε3) of the IgE molecule, preventing free IgE from binding to its receptors and reducing the release of bioactive mediators. Meanwhile, the reduction in serum free IgE leads to downregulation of FcεRI expression on the surface of effector cells, further inhibiting the response of effector cells to allergens. In controlled clinical studies, the correlation between free IgE levels and asthma symptoms indicates that greater inhibition of IgE may yield superior clinical benefits. Ligelizumab (QGE031), targeting human IgE, is another fully humanized monoclonal IgG1 antibody, which binds to the Cε3 domain of IgE with higher affinity than Omalizumab. Compared with Omalizumab, Ligelizumab exhibits a stronger and longer-lasting inhibitory effect on free IgE on the surfaces of mast cells and basophils, helping more effectively block the allergic cascade and improving clinical efficacy in the treatment of asthma. In addition to the two foregoing anti-IgE monoclonal antibodies, current therapeutic drugs for allergic asthma also ccomprise antihistamines, leukotriene receptor antagonists, β2-adrenergic receptor agonists, and glucocorticoids. These drugs provide temporary relief only by inhibiting inflammatory responses and have certain side effects.

Therefore, there is an urgent need in the art to develop a more effective, safe, and long-lasting method for treating or preventing allergic asthma and other allergic diseases.

### CONTENT OF THE INVENTION

An object of the present disclosure is to provide a more effective, safe, and long-lasting method for treating or preventing allergic asthma and other allergic diseases.

In one aspect of the present disclosure, an expression cassette is provided. The expression cassette comprises the following elements: a promoter, optionally a nucleotide sequence encoding a first signal peptide, a nucleotide sequence encoding a heavy chain of an anti-IgE antibody, optionally a nucleotide sequence encoding a linker, optionally a nucleotide sequence encoding a second signal peptide, and a nucleotide sequence encoding a light chain of an anti-IgE antibody.

In another optional embodiment, the linker comprises one or more selected from the group consisting of a self-cleaving linker, a Furin cleavage site, and an IRES linker.

In another optional embodiment, the anti-IgE antibody is selected from the group consisting of Ligelizumab, Omalizumab, or a combination thereof.

In another optional embodiment, the expression cassette comprises a structure of Formula I from the 5' end to the 3' end:

Z1-Z2-Z3-Z4-Z5-Z6-Z7-Z8-Z9 (Formula I)

wherein each "-" is independently selected from a bond or a nucleotide linking sequence;
Z1 is a promoter;
Z2 optionally is a nucleotide sequence encoding the first signal peptide;
Z3 is a nucleotide sequence encoding the heavy chain of an anti-IgE antibody;
Z4 is a Furin cleavage site;
Z5 is a nucleotide sequence encoding a self-cleaving linker;
Z6 optionally is a nucleotide sequence encoding a second signal peptide ; and
Z7 is a nucleotide sequence encoding the light chain of an anti-IgE antibody;
Z8 optionally is a woodchuck hepatitis virus posttranscriptional regulatory element (WPRE element);
Z9 optionally is a polyadenylation signal (polyA).

In another optional embodiment, the expression cassette comprises a structure of Formula II from the 5' end to the 3' end:

Z1-Z2-Z3-Z4'-Z6-Z7-Z8-Z9 (Formula I)

wherein Z1, Z2, Z3, Z6, Z7, Z8, and Z9 are defined as above, and Z4' is an IRES element;
each "-" is independently selected from a bond or a nucleotide linking sequence.

In another optional embodiment, the promoter is an enhanced promoter, comprising: CMV, CBH, EF1α, CAG, CAGG, CASI, desmin, TMCK, MCK, MHCK7, PGK, or TTR.

In another optional embodiment, a nucleotide sequence of the CBH promoter has ≥ 95% identity to the nucleotide sequence shown in SEQ ID NO.1, optionally ≥ 98%, or optionally ≥ 99%.

In another optional embodiment, the nucleotide sequence of the CBH promoter is shown in SEQ ID NO.: 1.

In another optional embodiment, the first signal peptide and the second signal peptide are each independently selected from the group consisting of SAP signal peptide, IHC signal peptide, ILC signal peptide, AP signal peptide, CSP signal peptide, IL2 signal peptide, TPA signal peptide, L1 signal peptide, H7 signal peptide, or a combination thereof.

In another optional embodiment, the nucleotide sequence of a heavy chain of an anti-IgE antibody is shown in SEQ ID NO: 2, or has 90% or more (optionally 95% or more, 96% or more, 97% or more, 98% or more, or 99% or more) sequence identity thereto.

In another optional embodiment, an amino acid sequence of the heavy chain of the anti-IgE antibody is shown in SEQ ID NO.: 3, or has 90% or more (optionally 95% or more, 96% or more, 97% or more, 98% or more, or 99% or more) sequence identity thereto.

In another optional embodiment, the nucleotide sequence of a light chain of an anti-IgE antibody is shown in SEQ ID NO:4, or has 90% or more (optionally 95% or more, 96% or more, 97% or more, 98% or more, or 99% or more) sequence identity thereto.

In another optional embodiment, an amino acid sequence of the light chain of the anti-IgE antibody is shown in SEQ ID NO:5, or has 90% or more (optionally 95% or more, 96% or more, 97% or more, 98% or more, or 99% or more) sequence identity thereto.

In another optional embodiment, the nucleotide sequence encoding the heavy chain of the anti-IgE antibody is a codon-optimized nucleotide sequence.

In another optional embodiment, the nucleotide sequence encoding the light chain of the anti-IgE antibody is a codon-optimized nucleotide sequence.

In another optional embodiment, the self-cleaving linker is selected from the group consisting of T2A, F2A, E2A, P2A, or a combination thereof.

In another optional embodiment, the poly(A) is a SV40 polyadenylation signal, and its nucleotide sequence has ≥ 95% identity to the nucleotide sequence shown in SEQ ID NO.: 6, optionally ≥ 98%, or optionally ≥ 99%.

In another optional embodiment, the nucleotide sequence of the poly(A) is shown in SEQ ID NO.: 6.

In another optional embodiment, the length of each nucleotide linking sequence is 0-30 nt, optionally 1-15 nt.

In another optional embodiment, the nucleotide sequence of the expression cassette is selected from the group consisting of:
(a) a nucleotide sequence shown in SEQ ID NO:7;
(b) a nucleotide sequence having ≥ 95% (optionally ≥ 98%, or optionally ≥ 99%) sequence identity to the nucleotide sequence shown in SEQ ID NO:7; and
(c) a nucleotide sequence complementary to the nucleotide sequence described in (a) or (b).

In another optional embodiment, the nucleotide sequence of the expression cassette comprises a DNA sequence, a cDNA sequence, or an mRNA sequence.

In another optional embodiment, the nucleotide sequence of the expression cassette comprises a single-stranded sequence or a double-stranded sequence.

In another aspect of the present disclosure, a vector is provided. The vector comprises an expression cassette described in any one of the foregoing aspects and embodiments.

In another optional embodiment, the vector comprises one or more promoters that are operably linked to a nucleotide sequence, enhancer, intron, terminator, polyadenylation sequence, origin of replication, selectable marker, nucleic acid restriction site, and/or homologous recombination site.

In another optional embodiment, the vector is selected from a plasmid or a viral vector.

In another optional embodiment, the vector is selected from the group consisting of lentiviral vector, adenoviral vector, adeno-associated virus vector (AAV), DNA virus vector, retroviral vector, or a combination thereof.

In another optional embodiment, the vector is an adeno-associated virus (AAV) vector.

In another optional embodiment, the vector is an AAV vector comprising or being engineered to carry nucleotide sequences encoding a heavy chain and a light chain of an anti-IgE antibody.

In another optional embodiment, the vector is used for expressing a heavy chain and a light chain of an anti-IgE antibody.

In another aspect of the present disclosure, an adeno-associated virus vector is provided. The adeno-associated virus vector comprises the expression cassette described in any one of the foregoing aspects and embodiments.

In another optional embodiment, the adeno-associated virus is selected from AAV6 or AAV8.

In another optional embodiment, the adeno-associated virus vector is used for treating allergic diseases.

In another optional embodiment, the allergic diseases are selected from the group consisting of allergic asthma, chronic spontaneous urticaria, or a combination thereof.

In another optional embodiment, the adeno-associated virus vector comprises a structure of Formula III:

A1-Z1-Z2-Z3-Z4-Z5-Z6-Z7-Z8-Z9-A2 (Formula III)

wherein,
each "-" is independently selected from a bond or a nucleotide linking sequence;
A1 is an L-ITR sequence;
Z1 is a promoter;
Z2 optionally is a nucleotide sequence encoding a first signal peptide;
Z3 optionally is a nucleotide sequence encoding a heavy chain of an anti-IgE antibody;
Z4 is a Furin cleavage site;
Z5 is a nucleotide sequence encoding a self-cleaving linker;
Z6 optionally is a nucleotide sequence encoding a second signal peptide; and
Z7 is a nucleotide sequence encoding a light chain of an anti-IgE antibody;
Z8 optionally is a woodchuck hepatitis virus posttranscriptional regulatory element (WPRE element);
Z9 optionally is a polyadenylation signal (polyA);
A2 is an R-ITR sequence.

In another optional embodiment, the adeno-associated virus vector comprises a structure of Formula IV:

A1-Z1-Z2-Z3-Z4'-Z6-Z7-Z8-Z9-A2 (Formula IV)

wherein,
each "-" is independently selected from a bond or a nucleotide linking sequence;
A1, Z1, Z2, Z3, Z6, Z7, Z8, Z9, and A2 are defined as above, and Z4' is an IRES element.

In another optional embodiment, a nucleotide sequence of the L-ITR is shown in SEQ ID NO.:8.

In another optional embodiment, a nucleotide sequence of the R-ITR is shown in SEQ ID NO.: 9.

In another optional embodiment, a nucleotide sequence of the adeno-associated virus backbone vector comprises the sequence shown in SEQ ID NO.: 10 (i.e., without the inserted target gene).

In another optional embodiment, the sequence of the adeno-associated virus vector is shown in SEQ ID NO.:11 (i.e., with an inserted target gene).

In another aspect of the present disclosure, a host cell is provided. The host cell comprises a vector or an adeno-associated virus vector described in any one of the foregoing aspects and embodiments, or a chromosome thereof carrying an exogenous expression cassette described in any one of the foregoing aspects and embodiments.

In another optional embodiment, the host cell is derived from a mammal, and the mammal is selected from humans or non-human mammals.

In another optional embodiment, the host cell is selected from the group consisting of 293T cells, 293 cells, or a combination thereof.

In another aspect of the present disclosure, provided is a use of the vector, adeno-associated virus vector, or host cell, described in any one of the foregoing aspects and embodiments, in the preparation of a formulation or composition for treating allergic diseases.

In another optional embodiment, the allergic diseases are selected from the group consisting of allergic asthma, chronic spontaneous urticaria, or a combination thereof.

In another aspect of the present disclosure, a pharmaceutical formulation is provided. The formulation comprises (a) the vector described, adeno-associated virus vector, or host cell described in any one of the foregoing aspects and embodiments, and (b) a pharmaceutically acceptable carrier or excipient.

In another optional embodiment, the dosage form of the pharmaceutical formulation is selected from the group consisting of lyophilized formulation, liquid formulation, or a combination thereof.

In another optional embodiment, the content of the vector in the pharmaceutical formulation is 1×10⁹-1×10¹⁶ viruses/milliliter, optionally 1×10¹²-1×10¹⁴ viruses/milliliter.

In another optional embodiment, the pharmaceutical formulation is used for treating allergic diseases.

In another optional embodiment, the allergic diseases are selected from the group consisting of allergic asthma, chronic spontaneous urticaria, or a combination thereof.

In another optional embodiment, the pharmaceutical formulation may be used as a monotherapy or as a combination therapy for the treatment of allergic diseases.

In another optional embodiment, the combination therapy comprises co-administering the pharmaceutical formulation with other drugs for treating allergic diseases.

In another aspect of the present disclosure, a treatment method is provided. The method comprises administering to a subject in need of the vector, adeno-associated virus vector, or host cell described in any one of the foregoing aspects and embodiments.

In another optional embodiment, the subject in need is selected from humans or non-human mammals.

In another optional embodiment, the non-human mammals are selected from rodents or primates, optionally mice, rats, rabbits, and monkeys.

In another optional embodiment, the administration dose of the vector, adeno-associated virus vector, or host cell described in any one of the foregoing aspects and embodiments is 1×10¹²-1×10¹⁴ per kilogram of body weight, optionally 5×10¹²-5×10¹³ per kilogram of body weight.

In another optional embodiment, the administration frequency of the vector described, adeno-associated virus vector, or host cell described in any one of the foregoing aspects and embodiments is a single dose.

Another aspect of the present disclosure provides a method for preparing a recombinant adeno-associated virus vector, comprising culturing the host cell described in any one of the foregoing aspects and embodiments to obtain the recombinant adeno-associated virus vector.

In another aspect of the present disclosure, a kit is provided, comprising:
(i) a first container and an active ingredient (a) in the first container, wherein the active ingredient (a) is selected from the group consisting of the vector, adeno-associated virus vector, host cell, or the pharmaceutical formulation described in any one of the foregoing aspects and embodiments;
(ii) optionally a second container and an active ingredient (b) in the second container, wherein the active ingredient (b) is selected from another drug for treating allergic diseases or an active ingredient thereof.

In another optional embodiment, the kit further comprises a package insert, wherein the package insert comprises instructions for administering the active ingredient (a) and (b) for treating allergic diseases.

In another optional embodiment, the first container and the second container are the same or different containers.

It should be understood that within the scope of the present disclosure, the above technical features of the present disclosure and the technical features specifically described below (e.g., the examples) may be combined with each other to form new or optional technical solutions. Due to space limitations, detailed descriptions are not provided here individually.

### DESCRIPTION OF THE DRAWINGS

FIG. 1 shows the electrophoresis analysis of the Ligelizumab-pUC plasmid.
FIG. 2 shows the enzyme digestion electrophoresis analysis of the Ligelizumab-pUC plasmid.
FIG. 3 shows the enzyme digestion electrophoresis analysis of the pAAV-CBH-MCS plasmid.
FIG. 4 shows the PCR identification electrophoresis analysis of the bacterial culture.
FIG. 5 shows the plasmid map of pAAV-CBH-MCS of the present disclosure.
FIG. 6 shows the design of the expression framework of the nucleic acid construct expressing Ligelizumab.
FIG. 7A shows the design of the nucleic acid construct (adeno-associated virus vector) expressing Ligelizumab.
FIG. 7B shows the map of the nucleic acid construct expressing Ligelizumab.
FIG. 8 shows the results of Western Blot detection of ligelizumab expression after transduction of cells with the adeno-associated virus expressing ligelizumab.
FIG. 9 shows the binding of purified ligelizumab to IgE.
FIG. 10 shows the change trend of the antibody concentration in the serum of BALB/c mice after receiving the adeno-associated virus expressing ligelizumab.
FIG. 11 shows the number of total leukocytes in the bronchoalveolar lavage fluid of mice.
FIG. 12 shows the number of eosinophils in the bronchoalveolar lavage fluid of mice.
FIG. 13 shows the plasmid map of pKL-lenti-kan-CBH-WPRE.
FIG. 14 shows the lentiviral gene therapy vector for the human IgE-neutralizing antibody ligelizumab.
FIG. 15 shows the expression of ligelizumab in the supernatant after transduction of cells with the lentivirus as detected by SDS-PAGE, where lane 1 represents the cell supernatant expressing ligelizumab; lane 2 represents the flow-through of the cell supernatant; lane 3 represents 5 µg of purified ligelizumab (sample added to loading buffer without β-mercaptoethanol); and lane 4 represents 5 µg of purified ligelizumab (sample added to loading buffer comprising β-mercaptoethanol).

### SPECIFIC IMPLEMENTATIONS

After extensive and in-depth research and numerous screenings, the applicant has developed, for the first time, an adeno-associated virus vector for the effectively treatment of allergic diseases via gene therapy. Specifically, the present disclosure provides an expression cassette for gene therapy of allergic diseases, which may be used for the gene therapy of allergic diseases, such as chronic spontaneous urticaria and allergic asthma. The expression cassette enables more efficient and cost-effective production of adeno-associated virus vectors for the gene therapy of allergic diseases. Experimental results show that the gene expression framework of the adeno-associated virus vector of the present disclosure may realize high-level expression of a target gene, and the adeno-associated virus vector may be used for treating allergic conditions, such as allergic asthma and chronic spontaneous urticaria. On this basis, the applicant has completed the present disclosure.

### Definitions

The following terms are defined to facilitate understanding of the present disclosure. As used in the present disclosure, unless expressly specified otherwise herein, each of the following terms shall have the meaning set forth below. Additional definitions are provided throughout the application.

As used herein, the term "about" generally refers to a value or composition within an acceptable error range for a specific value or composition as determined by one of ordinary skill in the art. This range depends in part on the method of measurement or determination. For example, as used herein, the expression "about 100" includes all values between 99 and 101 (e.g., 99.1, 99.2, 99.3, 99.4, etc.).

As used herein, the terms "containing" or "including (comprising)" may be open-ended, semi-closed, or closed. In other words, the terms also encompass "consisting essentially of" or "consisting of".

Sequence identity is determined by comparing two aligned sequences along a predetermined comparison window (which may be 50%, 60%, 70%, 80%, 90%, 95%, or 100% of the length of the reference nucleotide sequence or protein), and calculating the number of positions where identical residues occur. Typically, this is expressed as a percentage. Methods of measuring sequence identity of sequence identity of nucleotide sequences are well known to those skilled in the art.

As used herein, the terms "subject" and "subject in need" refer to any mammal or non-mammal. Mammals include, but are not limited to, humans and vertebrates, such as rodents, non-human primates, cattle, horses, dogs, cats, pigs, sheep, and goats.

### Ligelizumab

Ligelizumab, targeting human IgE, is a fully humanized monoclonal IgG 1 antibody, which binds to the Cε3 domain of IgE. Ligelizumab exhibits a stronger and longer-lasting inhibitory effect on free IgE on the surface of mast cells and basophils, helping more effectively block the allergic cascade and improving the clinical efficacy in the treatment of asthma. The antibody comprises the heavy chain and light chain of Ligelizumab.

### Furin Cleavage Site

In the present disclosure, a Furin cleavage site refers to an amino acid sequence motif that may be cleaved by Furin (a furin protease). A typical Furin cleavage site is Arg-X-(Arg/Lys)-Arg, and Furin protease cleaves the protein downstream of this typical Furin cleavage site. Furin-2A-mediated vector cleavage is highly efficient, with advantages such as high self-cleavage efficiency and ensuring equal expression levels of the light and heavy chains within the same open reading frame.

### IRES

In the present disclosure, "IRES linker" and "IRES" are used interchangeably, both referring to the internal ribosome entry site (IRES). Located at the 5' end of a gene, it is a short RNA sequence (about 150-250 bp). Such RNA sequences may fold into a structure similar to that of initiator tRNA, thereby mediating ribosomal RNA binding and initiating protein translation. IRES elements have the function of independently recruiting ribosomes without relying on a cap structure, and thus may initiate the translation of downstream genes.

### Nucleic Acid Encoding Sequence

In the present disclosure, an AAV viral vector expressing the heavy chain and light chain of an anti-IgE antibody is provided.

Exemplarily, an AAV viral vector expressing the heavy chain and light chain of Ligelizumab is provided in the present disclosure, with its nucleotide sequence shown in SEQ ID NO.:11. In another optional embodiment, the nucleotide sequence has ≥ 95% identity to the nucleotide sequence shown in SEQ ID NO.: 11, optionally ≥ 98%, and more optionally ≥ 99%.

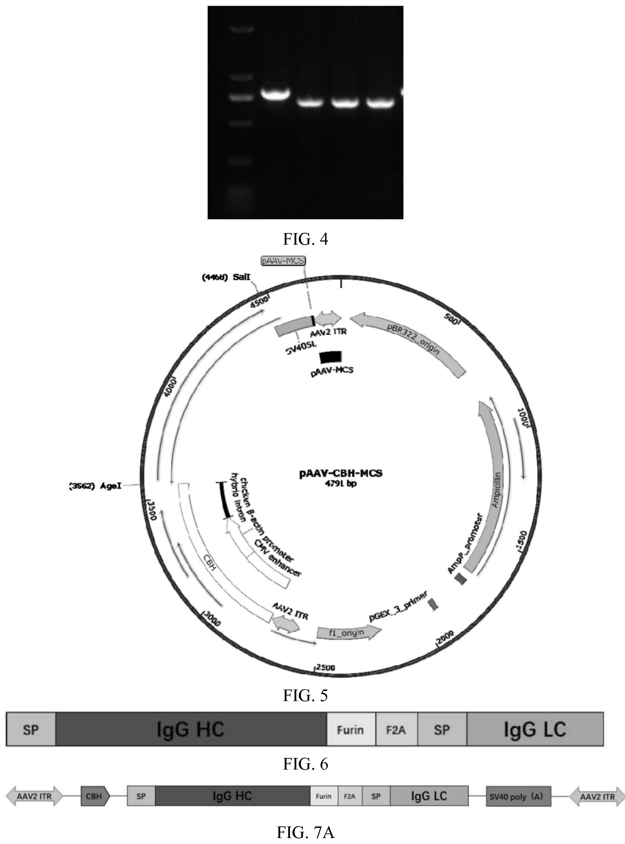

The polynucleotides of the present disclosure may be in the form of DNA or RNA. In another optional embodiment, the nucleotide is DNA. The DNA form includes cDNA, genomic DNA, or artificially synthesized DNA. DNA may be single-stranded or double-stranded. DNA may be a coding strand or a non-coding strand. The AAV viral vector of the present disclosure expresses the heavy chain and light chain of Ligelizumab, with the amino acid sequence of the heavy chain shown in SEQ ID NO.: 3 and its nucleotide sequence shown in SEQ ID NO.: 2, the amino acid sequence of the light chain shown in SEQ ID NO.5 and its nucleotide sequence shown in SEQ ID NO.4, and the structure shown in FIG. 6.

The nucleotide sequence may be DNA, RNA, cDNA, or PNA. The nucleotide sequence may be genomic, recombinant, or synthetic. The nucleotide sequence may be isolated or purified. The nucleotide sequence may be single-stranded or double-stranded. Optionally, the nucleotide sequence will encode the heavy chain and light chain of Ligelizumab as described herein. The nucleotide sequence may be derived by cloning, for example, using standard molecular cloning techniques including restriction enzyme digestion, ligation, and gel electrophoresis, as described in Sambrook *et al.,* Molecular Cloning: A laboratory manual, Cold Spring Harbour Laboratory Press. The nucleotide sequence may be isolated, for example, using PCR technology. Isolation refers to separating a nucleotide sequence from any impurities and other naturally associated nucleotide sequences and/or proteins. Optionally, it may also be free of cellular material, culture medium, or other chemicals from the purification or production process. The nucleotide sequence may be synthetic, such as produced by direct chemical synthesis, and may be provided as a naked nucleotide or in complex with proteins or lipids.

The full-length nucleotide sequence or its fragments of the present disclosure may generally be obtained by PCR amplification, recombination, or artificial synthesis. For PCR amplification, primers may be designed based on the disclosed relevant nucleotide sequences, especially the open reading frame (ORF) sequences. The relevant sequences may be amplified using commercially available cDNA libraries or cDNA libraries prepared by conventional methods known to those skilled in the art as templates. When the sequence is long, it is often necessary to perform two or more rounds of PCR amplification, followed by the correct assembly of the fragments from each round. Currently, the DNA sequence encoding the polypeptide (or its fragments or derivatives) of the present disclosure may be completely obtained by chemical synthesis. The DNA sequence may then be introduced into various existing DNA molecules (or vectors, for example) and cells known in the art.

The present disclosure also relates to vectors comprising the polynucleotides of the present disclosure, and host cells genetically engineered using the vectors or polypeptide-encoding sequences of the present disclosure. The foregoing polynucleotides, vectors, or host cells may be isolated.

As used herein, the term "isolated" refers to a substance that has been separated from its original environment (for naturally occurring substances, this means their natural environment). For example, polynucleotides and polypeptides in their natural state within living cells are not isolated and purified. However, the same polynucleotides or polypeptides are considered isolated and purified when separated from other substances present in their natural state.

Once the relevant sequences are obtained, they may be obtained in large quantities by recombinant methods. This typically involves cloning the relevant sequences into a vector, transferring the vector into a cell, and then isolating the relevant sequences from the proliferated host cells using conventional methods.

In addition, the relevant sequences may also be synthesized using artificial synthesis, particularly when the length of the fragment is short. Typically, fragments with very long sequences may be obtained by first synthesizing multiple small fragments and then ligating them together.

The method of amplifying DNA/RNA using PCR technology is optionally used to obtain the genes of the present disclosure. Primers for PCR may be appropriately selected based on the sequence information disclosed herein and may be synthesized using conventional methods. The amplified DNA/RNA fragments may be isolated and purified by conventional methods such as gel electrophoresis.

The present disclosure also relates to vectors comprising the polynucleotides of the present disclosure, and host cells genetically engineered using the vectors or protein-encoding sequence of the present disclosure, and methods for expressing the heavy chain and light chain of Ligelizumab using the host cells via recombinant techniques.

Using conventional recombinant DNA technology, host cells (e.g., mammalian cells) expressing the heavy chain and light chain of Ligelizumab of the present disclosure may be obtained by using the polynucleotide sequences of the present disclosure. Generally, the steps include introducing the expression cassette, vector, or adeno-associated virus vector described in any one of the foregoing aspects and embodiments into host cells.

Methods well known to those skilled in the art may be used to construct expression vectors containing the DNA sequences encoding the polypeptides of the present disclosure and appropriate transcriptional/translational control signals. These methods include in vitro recombinant DNA technology, DNA synthesis technology, in vivo recombination technology, etc. The aforementioned DNA sequences may be operably linked to appropriate promoters in the expression vectors to direct mRNA synthesis. The expression vectors also comprise ribosome binding sites for translation initiation and transcription terminators.

In addition, the expression vectors optionally comprise one or more selectable marker genes to provide phenotypic traits for selecting transformed host cells, such as dihydrofolate reductase, neomycin resistance, and green fluorescent protein (GFP) for eukaryotic cell culture, or tetracycline or ampicillin resistance for *Escherichia coli.*

Vectors comprising the foregoing appropriate DNA sequences and appropriate promoters or control sequences, may be used to transform appropriate host cells to enable to express the polypeptides.

Host cells may be prokaryotic cells, lower eukaryotic cells, or higher eukaryotic cells, such as mammalian cells (including human cells and non-human mammalian cells), such as CHO, NS0, COS7, or 293 cells. In an optional embodiment of the present disclosure, 293T cells or 293 cells are selected as the host cells.

Transformation of host cells with recombinant DNA may be performed using conventional techniques well known to those skilled in the art. When the host is a prokaryote such as *Escherichia coli,* competent cells capable of taking up DNA may be harvested after the exponential growth phase and treated by the CaCl₂ transformation method, as is known in the art. Another method is to use MgCl₂. If necessary, transformation may also be performed by electroporation. When the host is a eukaryote, the following DNA transfection methods may be selected: calcium phosphate co-precipitation method, and conventional mechanical methods, such as microinjection, electroporation, and liposome encapsulation.

The obtained transformants may be cultured by conventional methods to express the protein encoded by the gene of the present disclosure. Depending on the host cells used, the culture medium used in the culture may be selected from various conventional media. Cultivation is carried out under conditions suitable for the growth of the host cells. After the host cells have grown to an appropriate cell density, the selected promoter is induced by a suitable method (e.g., temperature shift or chemical induction), and the cells are cultured for an additional period of time.

The polypeptide in the foregoing method may be expressed intracellularly, on the cell membrane, or secreted extracellularly. If necessary, the protein can be isolated and purified by various separation methods utilizing its physical, chemical, and other properties. These methods are well known to those skilled in the art. Examples of those methods include, but are not limited to, conventional refolding treatment, treatment with protein precipitants (salting-out method), centrifugation, osmotic lysis, ultrasonication, ultracentrifugation, molecular sieve chromatography (gel filtration), adsorption chromatography, ion exchange chromatography, high-performance liquid chromatography (HPLC), and various other liquid chromatography techniques, and combinations of these methods.

### Adeno-associated Virus

Due to the characteristics of adeno-associated virus (AAV), such as smaller size compared with other viral vectors, non-pathogenicity, and the ability to transfect both dividing and non-dividing cells, AAV vector-based gene therapy methods targeting the eye, particularly hereditary retinal degenerative diseases, have attracted widespread attention.

Adeno-associated virus (AAV) belongs to the genus *Dependoparvovirus* of the family *Parvoviridae.* It is a type of single-stranded DNA defective virus with the simplest structure discovered to date, and its replication requires the participation of a helper virus (usually an adenovirus). It encodes the cap and rep genes within the inverted terminal repeats (ITRs) at both ends. ITRs play a decisive role in viral replication and packaging. The cap gene encodes the viral capsid proteins, and the rep gene is involved in viral replication and integration. AAV may infect a variety of cells.

Recombinant adeno-associated virus vectors (rAAVs) are derived from non-pathogenic wild-type adeno-associated virus. Due to their advantages including good safety, broad host cell range (dividing and non-dividing cells), low immunogenicity, and long-term expression of exogenous genes in vivo, rAAVs are regarded as one of the most promising gene transfer vectors and have been widely used in gene therapy and vaccine research worldwide. After more than a decade of research, the biological characteristics of recombinant adeno-associated virus have been deeply elucidated, and abundant data have been accumulated, especially regarding their application effects in various cells, tissues, and in vivo experiments. In medical research, rAAVs have been used in the study of gene therapy for various diseases (including in vivo and in vitro experiments); meanwhile, as a distinctive gene transfer vector, they are also widely applied in gene function research, construction of disease models, preparation of gene knockout mice, etc.

In an optional embodiment of the present disclosure, the vector is a recombinant AAV vector. AAV is a relatively small DNA virus, which may integrate into the genome of infected cells in a stable and site-specific manner. They may infect a wide range of cells without exerting any impact on cell growth, morphology, or differentiation, and they do not appear to be associated with human pathology. The AAV genome has been cloned, sequenced, and characterized. AAV comprises inverted terminal repeat (ITR) regions of about 145 bases at each end, which act as the origin of viral replication. The remainder of the genome is divided into two major regions with encapsidation functions: the left portion of the genome containing the rep gene involved in viral replication and gene expression; and the right portion of the genome containing the cap gene encoding the viral capsid proteins.

AAV vectors can be prepared using standard methods in the art. Any serotype of adeno-associated virus is suitable. Methods for purifying the vectors may be found in, for example, U.S. Patent Nos. 6566118, 6989264, and 6995006, the entire contents of disclosures of which are incorporated herein by reference. The preparation of hybrid vectors is described in, for example, PCT Application No. PCT/US2005/027091, the entire contents of disclosure of which are incorporated herein by reference. The use of AAV-derived vectors for in vitro and in vivo gene delivery has been described (see, for example, International Patent Application Publication Nos. WO91/18088 and WO93/09239; U.S. Patent Nos. 4,797,368, 6,596,535, and 5,139,941; and European Patent No. 0488528, all the entire contents of disclosure of which are incorporated herein by reference in their entirety). These patents describe various AAV-derived constructs which the rep and/or cap genes are deleted and replaced with a gene of interest, as well as the use of these constructs for delivering the gene of interest in vitro (into cultured cells) or in vivo (directly into an organism). Replication-defective recombinant AAV may be prepared by co-transfecting the following plasmids into a cell line infected with a human helper virus (e.g., adenovirus): a plasmid comprising the nucleotide sequence of interest flanked by two AAV inverted terminal repeat (ITR) regions, and a plasmid carrying the AAV encapsidation genes (rep and cap genes). The resulting AAV recombinants are then purified by standard techniques.

In some embodiments, the recombinant vector is encapsidated into viral particles, such as AAV viral particles including, but not limited to, AAV1, AAV2, AAV3, AAV4, AAV5, AAV6, AAV7, AAV8, AAV9, AAV10, AAV11, AAV12, AAV13, AAV14, AAV15, and AAV16. Thus, the present disclosure includes recombinant viral particles (recombinant by virtue of containing a recombinant polynucleotide) comprising any of the vectors described herein. Methods for producing such particles are known in the art and are described in U.S. Patent No. 6,596,535.

### Expression Vectors and Host Cells

In the present disclosure, an expression vector for expressing the heavy chain and light chain of Ligelizumab is also provided.

With the provided sequence information, skilled technicians may use available cloning techniques to generate nucleotide sequences or vectors suitable for transduction into cells.

Optionally, nucleotide sequences encoding the heavy chain and light chain of Ligelizumab are provided as vectors, optionally expression vectors. Optionally, they may be provided as gene therapy vectors that are optionally suitable for transduction and expression in 293T cells. The vectors may be viral or non-viral (such as plasmids). Viral vectors comprise those derived from the following: adenoviruses, adeno-associated viruses (AAVs) including mutated forms, retroviruses, lentiviruses, herpesviruses, vaccinia viruses, MMLV, GaLV, simian immunodeficiency virus (SIV), HIV, poxviruses, and SV40. Preferably, the viral vectors are replication-defective, although replication-deficient, replication-competent, or conditionally replication-competent vectors are also contemplated. Viral vectors may typically remain in an extrachromosomal state without integrating into the genome of 293T cells. The optional viral vector for introducing nucleotide sequences encoding the heavy chain and light chain of Ligelizumab into 293T cells is an AAV vector, such as a self-complementary adeno-associated virus (scAAV). Selective targeting may be achieved using specific AAV serotypes (AAV serotype 2 to AAV serotype 12) or modified versions of any of these serotypes (including AAV 4YF and AAV 7m8 vectors).

In another optional embodiment, the adeno-associated virus used in the present disclosure is AAV8; optionally, the adeno-associated virus is single-stranded AAV8.

Viral vectors may be modified to delete any non-essential sequences. For example, in AAV, the virus may be modified to delete all or part of the IX gene, Ela gene, and/or Elb gene. For wild-type AAV, replication is highly inefficient in the absence of a helper virus such as adenovirus. For recombinant adeno-associated viruses, optionally, the replication genes and capsid genes are provided in trans (in the pRep/Cap plasmid), and only the 2ITRs of the AAV genome are retained and packaged into virions, while the required adenoviral genes are provided by adenovirus or another plasmid. Similar modifications may be made to lentiviral vectors.

Viral vectors have the ability to enter cells. However, non-viral vectors such as plasmids may be complexed with agents to facilitate their uptake by target cells. Such agents include polycationic agents. Optionally, delivery systems such as liposome-based delivery systems may be used. The vectors for use in the present disclosure are optionally suitable for in vivo or in vitro use, and optionally suitable for use in humans.

The vector will optionally comprise one or more regulatory sequences to direct the expression of the nucleotide sequence in retinal target cells. The regulatory sequences may include promoters, introns, enhancers, transcription terminators, polyadenylation sequences, origins of replication, nucleic acid restriction sites, and homologous recombination sites that are operably linked to the nucleotide sequence. The vector may also comprise a selectable marker, for example, to determine the expression of the vector in a growth system (e.g., bacterial cells) or in retinal target cells.

As used herein, the term "operably linked" generally refers to a nucleotide sequence functionally related to the sequence to which it is operably linked, such that they are joined in a manner that allows them to influence each other's expression or function. For example, a nucleotide sequence operably linked to a promoter may have an expression pattern that is affected by the promoter.

A promoter mediates the expression of the nucleotide sequence linked to it. A promoter may be constitutive or may be inducible. A promoter may direct ubiquitous expression in inner retinal cells or neuron-specific expression. In the latter case, the promoter may direct cell type-specific expression, for example, retinal ganglion cells. Suitable promoters will be known to those skilled in the art. For example, suitable promoters may be selected from the group consisting of L7, thy-1, recoverin, calbindin, human CMV, GAD-67, chicken actin, hSyn, Grm6, and Grm6 enhancer-SV40 fusion protein. Targeting may be achieved using cell-specific promoters, such as Grm6-SV40 for selective targeting of retinal ganglion cells. The Grm6 promoter is a fusion of a 200-base pair enhancer sequence of the Grm6 gene and the SV40 eukaryotic promoter. Grm6 gene encodes the metabotropic glutamate receptor mGluR6 specific to retinal ganglion cells. Optional sources of the Grm6 gene are mice and humans. Ubiquitous expression may be achieved using pan-neuronal promoters, examples of which are known and available in the art. One such example is CAG. The CAG promoter is a fusion of the CMV early enhancer and the chicken actin promoter.

An example of a suitable promoter is the immediate early cytomegalovirus (CMV) promoter sequence. This promoter sequence is a strong constitutive promoter sequence capable of driving high-level expression of any polynucleotide sequence operably linked to it. Another example of a suitable promoter is elongation factor-1α (EF-1α). However, other constitutive promoter sequences may also be used, including, but not limited to, the simian virus 40 (SV40) early promoter, mouse mammary tumor virus (MMTV), human immunodeficiency virus (HIV) long terminal repeat (LTR) promoter, MoMuLV promoter, avian leukemia virus promoter, Epstein-Barr virus immediate early promoter, Rous sarcoma virus promoter, and human gene promoters such as, but not limited to, the actin promoter, myosin promoter, hemoglobin promoter, and creatine kinase promoter. Optionally, the present disclosure should not be limited to the use of constitutive promoters. Inducible promoters are also contemplated as part of the present disclosure. The use of an inducible promoter provides a molecular switch that may turn on the expression of a polynucleotide sequence operably linked to the inducible promoter when such expression is desired, or turn off the expression when it is not desired. Examples of inducible promoters include, but are not limited to, the metallothionein promoter, glucocorticoid promoter, progesterone promoter, and tetracycline promoter.

An example of a suitable promoter is the CBH promoter.

A variety of expression vectors may be used for the expression of the heavy chain and light chain of Ligelizumab in mammalian cells (optionally human cells, more preferably human 293T cells). Optionally, an adeno-associated virus is used as the expression vector in the present disclosure.

The present disclosure further provides a method for constructing a recombinant adeno-associated virus vector comprising the sequences encoding the heavy chain and light chain of Ligelizumab. The method may rapidly and conveniently construct a recombinant adeno-associated virus vector carrying the sequences encoding the heavy chain and light chain of Ligelizumab, and package it to obtain a replication-defective adeno-associated virus vector.

In another optional embodiment, the sequence of the adeno-associated virus vector carrying the sequences encoding the heavy chain and light chain of Ligelizumab described in the present disclosure is shown in SEQ ID NO.: 11.

### Gene Therapy Vectors

The gene therapy vectors in the present disclosure are viral expression vectors. According to the present disclosure, the viral expression vectors are adeno-associated virus (AAV) vectors, which are better suited for efficient transduction in tissues of interest. Upon transfection, AAV elicits only a mild immune response (if any) in the host. In an optional embodiment of the present disclosure, the gene therapy vector is an AAV serotype 8 vector.

Currently, recombinant adeno-associated virus (rAAV) is one of the important viral vectors in the field of gene therapy. Its safety and efficacy have been verified in numerous clinical trials, endowing it with broad application prospects. To develop an ideal gene therapy product for allergic diseases, a recombinant adeno-associated virus (rAAV) vector, characterized by a broad host range, the ability to infect both dividing and non-dividing cells, high viral titer, strong specificity, low toxicity and immunogenicity, non-integration into chromosomes, and capacity for long-term expression of exogenous genes, is selected. In this disclosure, the rAAV8 vector carrying the Ligelizumab-encoding gene was intramuscularly injected into IgE/FcεRI double-humanized BALB/c asthma model mice, which could significantly reduce the proportion of eosinophils in the total leukocytes in bronchoalveolar lavage fluid, while decreasing the secretion of Th2 cytokines, thereby alleviating asthma symptoms. In the present disclosure, a gene therapy drug for allergic diseases is provided, and to date, no relevant reports have been seen.

In the present disclosure, the AAV vectors are single-stranded AAV, and recombinant viral vectors may be produced according to standard techniques. For example, recombinant adeno-associated virus vectors may be packaged in human 293T cells (which provide trans E1A and E1B functions) to achieve a titer in the range of 10¹⁰-10¹³ viral particles per milliliter. Prior to in vivo application, the viral vectors may be purified by affinity purification and subsequent filtration. Purification reduces potential adverse effects in the subject administered the vectors. The administered virus is essentially free of wild-type virus and replication-competent virus. The purity of the virus can be demonstrated by suitable methods, such as sodium dodecyl sulfate-polyacrylamide gel electrophoresis (SDS-PAGE) followed by silver staining, or HPLC.

A suitable dose of AAV for human use is in the range of about 1×10¹²-1×10¹⁴ vg/kg.

The gene therapy vector may be suitable for administration by via injection.

**In** the present disclosure, a host cell for packaging the adeno-associated virus containing the genes encoding the heavy chain and light chain of Ligelizumab is provided. Optionally, the host cell is a mammalian cell (optionally a human cell, more preferably a human 293T cell). The cells or animals are then infected with the adeno-associated virus containing the genes encoding the heavy chain and light chain of Ligelizumab to achieve in vitro and in vivo expression of the Ligelizumab antibody.

In an optional embodiment, an adeno-associated virus vector for gene therapy of allergic diseases is provided. Experimental results show that the gene expression framework of the adeno-associated virus vector may realize high-level expression of a target gene, and the gene sequence construct may be used for treating allergic conditions such as allergic asthma and chronic spontaneous urticaria. The following are the cis-acting elements, origin of replication, and polyadenylation signal used in the present vector:
1. Cis-acting element CBH (CMV Enhancer + chicken β-actin promoter + hybrid intron), which is located upstream of the antibody gene sequence and participates in the regulation of antibody gene sequence expression. Experimental results indicate that this element may effectively enhance gene expression.
2. SV40 polyadenylation signal. Polyadenylation is a mechanism that promotes the covalent attachment of polyadenylic acid to messenger RNA (mRNA) molecules during protein biosynthesis. The polyadenylate tail (or polyA tail) protects mRNA from exonuclease attack and is crucial for transcription termination, nuclear export of mRNA, and subsequent translation.
3. pBR322_origin, which is the replication region sequence of plasmid pBR322. In *Escherichia coli,* plasmid replication initiation starts from this site.
4. Codon optimization of Ligelizumab. Codon optimization is an important step in gene expression optimization, involving aspects such as codon usage bias, rare codons, mRNA secondary structure, GC content distribution, and splice sites.

Experimental data of the present disclosure show that the adeno-associated virus vector pAAV-CBH-ligelizumab may be used for efficient gene therapy of allergic diseases, such as allergic asthma and chronic spontaneous urticaria.

### Formulations and Compositions

In the present disclosure, a formulation or composition is provided. The formulation or composition comprises (a) the vector, or adeno-associated virus vector described in any one of the foregoing aspects and embodiments, and (b) a pharmaceutically acceptable carrier or excipient.

In another optional embodiment, the pharmaceutical formulation is used for treating allergic diseases.

For the convenience of clinical application, the pharmaceutical composition of the present disclosure may be contained in an injectable administration device (e.g., an injection needle). The injectable administration device may contain a single dose of the pharmaceutical composition. The injectable administration device may be included in a kit for convenient storage and use. During transportation, the micro-container containing the drug suspension should be placed in dry ice. It should be stored in a -80°C refrigerator under normal conditions.

The kit or reagent kit described in the present disclosure may further include a package insert to enable those skilled in the art to use it in the correct manner.

The "active ingredient" in the pharmaceutical composition described in the present disclosure refers to the vector described in the present disclosure, such as viral vectors (including adeno-associated virus vectors). The "active ingredient", formulations, and/or compositions described in the present disclosure may be used for treating ocular diseases. The term "safe and effective amount" refers to an amount of the active ingredient sufficient to significantly improve the condition or symptoms without causing severe side effects. The term "pharmaceutically acceptable carrier or excipient" refers to one or more compatible solid or liquid fillers or gel materials that are suitable for human use and must be of sufficient purity and sufficiently low toxicity. The term "compatibility" refers to the components in the composition may be mixed with the active ingredient of the present disclosure and with each other without significantly reducing the efficacy of the active ingredient.

The composition may be liquid or solid, such as powder, gel, or paste. Optionally, the composition is a liquid, optionally an injectable liquid. Suitable excipients will be known to those skilled in the art.

In the present disclosure, the vector is provided as an injectable liquid. Optionally, the injectable liquid is provided in a capsule or syringe.

Some examples of pharmaceutically acceptable carriers comprise cellulose and its derivatives (e.g., sodium carboxymethylcellulose, sodium ethylcellulose, cellulose acetate, etc.), gelatin, talc, solid lubricants (e.g., stearic acid, magnesium stearate), calcium sulfate, vegetable oils (e.g., soybean oil, sesame oil, peanut oil, olive oil, etc.), polyols (e.g., propylene glycol, glycerol, mannitol, sorbitol, etc.), emulsifiers (e.g., Tween^{®}), wetting agents (e.g., sodium dodecyl sulfate), coloring agents, flavoring agents, stabilizers, antioxidants, preservatives, pyrogen-free water, etc.

The composition may comprise physiologically acceptable sterile aqueous or non-aqueous solutions, dispersions, suspensions, or emulsions, and sterile powders for reconstitution into sterile injectable solutions or dispersions. Suitable aqueous and non-aqueous carriers, diluents, solvents, or excipients include water, ethanol, polyols, and suitable mixtures thereof.

The nucleic acids or fused nucleic acids encoding the heavy chain and light chain of Ligelizumab provided in the present disclosure may produce the heavy chain and light chain of Ligelizumab in vitro or in vivo. Formulations containing the heavy chain and light chain of Ligelizumab may be used in the preparation of a medicament for treating allergic diseases.

### Method of Treatment

The present disclosure provides a method for treating allergic diseases. The method comprises introducing a vector comprising nucleotide sequences encoding the heavy chain and light chain of Ligelizumab into a body. The method may include administering the nucleic acid vector to the body.

The present disclosure provides a nucleic acid vector for use in a method of treating allergic diseases, wherein the nucleic acid vector comprises nucleotide sequences encoding the heavy chain and light chain of Ligelizumab. The composition of the present disclosure may be administered alone or in combination with other therapeutic agents (e.g., formulated in the same pharmaceutical composition).

As used herein, treating a disease means administering a nucleic acid or vector as described herein to ameliorate or alleviate one or more symptoms of the disease.

The method of the present disclosure comprises introducing nucleotide sequences encoding the heavy chain and light chain of Ligelizumab into the body. Optionally, the method comprises contacting a cell with a vector (preferably a virus, more preferably an adeno-associated virus) comprising nucleotide sequences encoding the heavy chain and light chain of Ligelizumab. Optionally, the cell is from an animal or a human. When nucleotide sequences are provided in multiple (two or more) doses, these doses may be separated by suitable time intervals, for example, 30 seconds to several hours, or one or more days.

Each dose may comprise an effective amount of the nucleotide sequence or viral vector. The effective dose of the nucleotide sequence or viral vector may range from 1×10¹²-1×10¹⁴ vg/kg per treatment regimen.

### Pharmaceutical Compositions and Kits

As used herein, the term "comprising" includes "containing", "consisting essentially of", and "consisting of'.

As used herein, the term "consisting essentially of" refers that, in addition to the effective active ingredients or auxiliary ingredients, the pharmaceutical composition may also contain minor ingredients and/or impurities that are present in small amounts and do not affect the effective ingredients.

For the convenience of clinical application, the pharmaceutical composition of the present disclosure may be contained in an injectable administration device (e.g., an injection needle). The injectable administration device may contain a single dose of the pharmaceutical composition. The injectable administration device may be included in a kit for convenient storage and use. During transportation, the micro-container containing the drug suspension should be placed in dry ice. It should be stored in a -80°C refrigerator under normal conditions.

The kit or reagent kit described in the present disclosure may further comprise a package insert to enable those skilled in the art to use it in the correct manner.

As used herein, the term "effective amount" or "effective dose" refers to an amount that is capable of exerting a functional or active effect on humans and/or animals and is acceptable to humans and/or animals.

The effective amount of the active ingredient described in the present disclosure may vary depending on factors such as the mode of administration and the severity of the disease to be treated. The selection of a optional effective amount may be determined by those of ordinary skill in the art based on various factors (e.g., through clinical trials). Such factors include, but are not limited to: the pharmacokinetic parameters of the active ingredient, such as bioavailability, metabolism, half-life, etc.; the severity of the disease to be treated in the patient, the patient's weight, the patient's immune status, the route of administration, etc. For example, depending on the urgency of the treatment condition, several divided doses may be administered daily, or the dose may be proportionally reduced.

As used herein, a "pharmaceutically acceptable" ingredient is a substance that is suitable for use in humans and/or mammals without excessive adverse side effects (e.g., toxicity), that is, with a reasonable benefit/risk ratio.

The term "pharmaceutically acceptable carrier" refers to a carrier used for the administration of therapeutic agents, comprising various excipients and diluents, that is, such pharmaceutical carriers that are not themselves essential active ingredients and do not exhibit excessive toxicity following administration.

Suitable pharmaceutically acceptable carriers are well known to those of ordinary skill in the art. A comprehensive description of pharmaceutically acceptable carriers is found in Remington's Pharmaceutical Sciences (Mack Pub. Co., N. J. 1991); pharmaceutically acceptable carriers in the composition may comprise liquids such as water, BBS (Balanced Salt Solution) phosphate buffer, Ringer's solution, normal saline, balanced salt solution, glycerol, or sorbitol, etc.

In addition, auxiliary substances may also be present in these carriers, such as lubricants, glidants, wetting or emulsifying agents, pH buffering substances, and stabilizers, etc.

In the embodiments of the present disclosure, the pharmaceutical composition is in a liquid dosage form; optionally, it is an injection.

The pharmaceutical composition of the present disclosure may be used alone or in combination in the treatment of allergic diseases. The foregoing combination use includes: combination with other drugs for treating allergic diseases. Such other drugs for treating allergic diseases comprise: antihistamines, leukotriene receptor antagonists, β2-adrenergic receptor agonists, and glucocorticoids, etc.

In addition, the present disclosure has created a gene therapy construct for allergic diseases based on a recombinant adeno-associated virus vector. The heavy chain and light chain of the anti-IgE-neutralizing antibody Ligelizumab are fused and expressed via F2A to form a simple and efficient expression framework. Experimental results show that the expression framework achieves high expression efficiency following delivery by a rAAV vector.

The object of the present disclosure is to provide a nucleic acid construct for gene therapy of allergic-related diseases, addressing issues in the prior art.

To achieve the above object, a nucleic acid construct comprising nucleotides encoding Ligelizumab is provided in the present disclosure.

The present application also provides an adeno-associated virus vector system comprising the aforementioned nucleic acid construct and a helper plasmid.

The present application further provides an adeno-associated virus that is generated by viral packaging of the nucleic acid construct.

The present application further provides a cell line that is required for the packaging of the adeno-associated virus.

The present application further provides the use of the foregoing nucleic acid construct, adeno-associated virus, adeno-associated virus vector system, and cell line, in the preparation of a product for preventing and treating related allergic diseases such as asthma.

As described above, the nucleic acid construct for gene therapy of allergic-related diseases of the present disclosure has the following beneficial effects: the present disclosure has created a nucleic acid construct comprising a Ligelizumab expression framework, which can be used for the efficient expression of active ligelizumab both in vitro and in vivo, providing a therapeutic idea for gene therapy drugs for allergic diseases such as allergic asthma and chronic spontaneous urticaria. The construct of the present disclosure may be delivered via viral or non-viral vectors and is applicable for the gene therapy of allergic asthma and related diseases.

In addition, a nucleic acid construct is provided in the present disclosure by tandemly linking the heavy chain and light chain of Ligelizumab via a cleavable 2A peptide, followed by subcloning into an rAAV expression vector. From the 5' to 3' end, the construct sequentially comprises: the 5' inverted terminal repeat (5'ITR) of AAV, a promoter, the heavy chain of the antibody (comprising a signal peptide), an F2A short peptide, the light chain of the antibody (comprising a signal peptide), WPRE, a polyadenylation signal (polyA), and the 3' inverted terminal repeat (3'ITR) of AAV. Among them, the promoter may be any one of CMV (GenBank: MN996867.1, 235 to 818), CBH (SEQ ID NO.: 1), EF1α (GenBank: KY447299.1, 529 to 1710), CAG (GenBank: KC152483.1, 200 to 1096), CAGG (GenBank: GU299216.1, 63 to 1664), CASI (GenBank: ON512571.1, 80 to 1104), desmin (GenBank: M63391.1, 2194 to 3233), TMCK (SEQ ID NO.: 12), MCK (SEQ ID NO.: 13), MHCK7 (SEQ ID NO.: 14), PGK (GenBank: JF313343.1, 683 to 1192), and TTR (SEQ ID NO.: 15). The signal peptide may be any one of SAP signal peptide (SEQ ID NO.: 16), IHC signal peptide (SEQ ID NO.: 17), ILC signal peptide (SEQ ID NO.: 18), AP signal peptide (SEQ ID NO.: 19), CSP signal peptide (SEQ ID NO.: 20), IL2 signal peptide (SEQ ID NO.: 21), TPA signal peptide (SEQ ID NO.: 22), L1 signal peptide (SEQ ID NO.: 23), and H7 signal peptide (SEQ ID NO.: 24). The 2A peptide linking the heavy chain and light chain is any one of T2A, F2A, E2A, and P2A. This construct is co-transfected into 293T cells together with two other AAV packaging plasmids, pAAV-RC and phelper, thereby generating an infectious rAAV vector. After purification, the viral vector may be administered to patients via intramuscular or intravenous injection to achieve long-term and sustained expression of Ligelizumab. The expressed antibody may neutralize allergenic IgE in patients, thereby exerting a therapeutic effect on allergic diseases such as allergic asthma and chronic spontaneous urticaria. In the present disclosure, the capsid used for AAV packaging may be derived from any one of AAV1, AAV2, AAV3, AAV4, AAV7, AAV8, AAV9, AAVrh.10, AAVDJ/8, and AAV6.2FF.

In the specific embodiment, antibody molecule is constructed, and cloned into an rAAV vector. In 293T cells, the vector is packaged into the corresponding adeno-associated virus, and 293T cells, as well as differentiated muscle cell lines, are infected with the virus at a specific biological titer. Quantitative and qualitative detection is performed on the antibody molecule produced in the cell supernatant. Meanwhile, normal BALB/c mice are intramuscularly injected with the virus at a specific biological titer to detect the expression of Ligelizumab. Finally, the adeno-associated virus is administered via intramuscular injection to IgE/FcεRI double-humanized BALB/c asthma model mice to determine the proportion of eosinophils in the total leukocytes in the bronchoalveolar lavage fluid.

The main advantages of the present disclosure include:
(1) The present disclosure provides, for the first time, a gene therapy adeno-associated virus vector capable of effectively treating allergic diseases. Specifically, the present disclosure provides an expression cassette for gene therapy of allergic diseases, which may be used for the gene therapy of allergic diseases such as chronic spontaneous urticaria and allergic asthma. The expression cassette is used to produce adeno-associated virus gene therapy vectors for the gene therapy of the foregoing allergic diseases with higher efficiency and lower cost. Experimental results show that the gene expression framework of the adeno-associated virus vector of the present disclosure may realize high-level expression of a target gene, and the adeno-associated virus vector is used for treating allergic conditions such as allergic asthma and chronic spontaneous urticaria.
(2) The present disclosure relates to in vivo antibody production. The traditional method of treatment involves recombinant expression of an anti-IgE antibody in vitro using genetic engineering techniques, followed by subcutaneous injection to neutralize allergenic IgE in the body and control symptoms. However, antibodies have a short half-life in vivo, requiring monthly injections that are not only costly but also inconvenient for patients. In the present disclosure, by constructing the anti-IgE antibody molecule into an rAAV vector, then being packaged into an infectious rAAV in vitro, and delivering it to a patient, the anti-IgE antibody may be produced continuously in vivo for a long period of time, achieving the effect of one-time administration with long-lasting efficacy.
(3) The present disclosure refers to a muscle tissue-specific expression vector. The viral vector packaged by the construct of the present disclosure achieve targeted expression in muscles. Since rAAV viral vectors are not integrated into the genome in cells, but exist as episomes, they are very prone to loss in cells with rapid differentiation or high proliferation rates, resulting in the loss of their corresponding function. In contrast, muscle cells almost never differentiate throughout their lifespan, a characteristic that makes them highly suitable for the long-term and sustained expression of rAAV gene therapy vectors, thereby enabling prolonged therapeutic effects.
(4) The present disclosure has created a nucleic acid construct containing a ligelizumab expression framework, which may be used for the efficient expression of active Ligelizumab both in vitro and in vivo, providing a therapeutic idea for gene therapy drugs for allergic diseases such as allergic asthma and chronic spontaneous urticaria. The construct of the present disclosure may be delivered via viral or non-viral vectors and is applicable for the gene therapy of allergic asthma and related diseases.
(5) In the present disclosure, the amino acid expression cassette of Ligelizumab has been optimized based on principles such as human codon usage bias, mRNA secondary structure, appropriate GC content (avoiding excessively high GC content), avoidance of various cis-elements involved in transcription and translation processes, avoidance of the appearance of rare codons, and avoidance of splice sites, significantly improving the expression level and stability of the target protein.

The present disclosure is further set forth below in conjunction with specific examples. It should be understood that these examples are only intended to illustrate the present disclosure and not to limit the scope of the present disclosure. For the experimental methods without specific conditions noted in the following examples, conventional conditions are generally followed, such as those described in Sambrook et al., Molecular Cloning: A Laboratory Manual (New York: Cold Spring Harbor Laboratory Press, 1989), or the conditions recommended by the manufacturer. Unless otherwise specified, percentages and parts are percentages by weight and parts by weight.

Unless otherwise specified, all materials and reagents in the specification of the present disclosure are commercially available products.

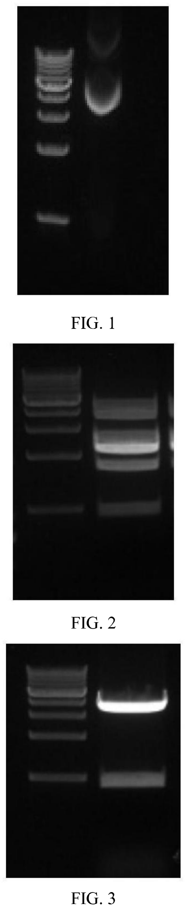

### Example 1: Construction of the rAAV8-Ligelizumab vector (a detailed description of the vector construction process, taking the structure in FIG. 3 as an example)

The entire construction process was specified as follows:
1. Synthesis of the IgE-neutralizing antibody Ligelizumab by GenScript
   Structure of Ligelizumab: From the N-terminus to the C-terminus of the protein molecule, it consists of: signal peptide of the heavy chain (taking the TPA signal peptide as an example, sequence: MDAMKRGLCCVLLLCGAVFVSP, SEQ ID NO.: 22) - heavy chain (HC) of the antibody molecule (taking Ligelizumab HC as an example) - Furin cleavage site - F2A self-cleaving 2A sequence -signal peptide of the light chain (taking the L1 signal peptide as an example, sequence: MDMRVPAQLLGLLLLWLSGARC, SEQ ID NO.: 23) - light chain (LC) of the antibody molecule (taking Ligelizumab LC as an example) (FIG. 6). The heavy chain (HC) and light chain (LC) molecules of the indicated sequence carry their respective signal peptide sequences, and the heavy chain (SEQ ID NO.2) and light chain (Sequence ID No: 4) of the antibody molecule are linked via a Furin cleavage site and a self-cleaving 2A sequence, wherein the sequences encoding the light chain and heavy chain of the antibody are codon-optimized sequences.
2. Upon receipt of the Ligelizumab gene-containing plasmid (Ligelizumab-pUC57) and bacterial culture, the bacterial culture harboring the plasmid was resuscitated in 5 mL of culture medium containing the corresponding antibiotic, followed by shaking incubation overnight at 37°C and 200 rpm.
3. The Ligelizumab-pUC57 plasmid was extracted. Plasmid electrophoresis analysis (Marker: 1kb DNA Ladder) is shown in FIG. 1.
4. Enzyme digestion of the target gene fragment and vector fragment.

1) Acquisition of the target gene. The Ligelizumab-pUC57 was subjected to AgeI-SalI-ScaI triple enzyme digestion to obtain the complete Ligelizumab structure. The sizes of the bands after enzyme digestion were 2.2 kb, 0.9 kb, and 1.8 kb. The 2.2 kb fragment was recovered. Enzyme digestion electrophoresis analysis (Marker: 1kb DNA Ladder) is shown in FIG. 2.
2) Acquisition of the vector fragment. The plasmid pAAV-CBH-MCS, with a structure shown in FIG. 5, was subjected to double enzyme digestion with AgeI and SalI. The sizes of the bands after enzyme digestion were 3.9 kb and 0.9 kb. The 3.9 kb fragment was recovered.

### pAAV-CBH-MCS nucleotide sequence:

ACATGTGAGCAAAAGGCCAGCAAAAGGCCAGGAACCGTAAAAAGGC
CGCGTTGCTGGCGTTTTTCCATAGGCTCCGCCCCCCTGACGAGCATCACAA
AAATCGACGCTCAAGTCAGAGGTGGCGAAACCCGACAGGACTATAAAGAT
ACCAGGCGTTTCCCCCTGGAAGCTCCCTCGTGCGCTCTCCTGTTCCGACCC
TGCCGCTTACCGGATACCTGTCCGCCTTTCTCCCTTCGGGAAGCGTGGCGC
TTTCTCATAGCTCACGCTGTAGGTATCTCAGTTCGGTGTAGGTCGTTCGCTC
CAAGCTGGGCTGTGTGCACGAACCCCCCGTTCAGCCCGACCGCTGCGCCTT
ATCCGGTAACTATCGTCTTGAGTCCAACCCGGTAAGACACGACTTATCGCC
ACTGGCAGCAGCCACTGGTAACAGGATTAGCAGAGCGAGGTATGTAGGCG
GTGCTACAGAGTTCTTGAAGTGGTGGCCTAACTACGGCTACACTAGAAGGA
CAGTATTTGGTATCTGCGCTCTGCTGAAGCCAGTTACCTTCGGAAAAAGAG
TTGGTAGCTCTTGATCCGGCAAACAAACCACCGCTGGTAGCGGTGGTTTTT
TTGTTTGCAAGCAGCAGATTACGCGCAGAAAAAAAGGATCTCAAGAAGAT
CCTTTGATCTTTTCTACGGGGTCTGACGCTCAGTGGAACGAAAACTCACGT
TAAGGGATTTTGGTCATGAGATTATCAAAAAGGATCTTCACCTAGATCCTTT
TAAATTAAAAATGAAGTTTTAAATCAATCTAAAGTATATATGAGTAAACTTG
GTCTGACAGTTACCAATGCTTAATCAGTGAGGCACCTATCTCAGCGATCTGT
CTATTTCGTTCATCCATAGTTGCCTGACTCCCCGTCGTGTAGATAACTACGAT
ACGGGAGGGCTTACCATCTGGCCCCAGTGCTGCAATGATACCGCGAGACCC
ACGCTCACCGGCTCCAGATTTATCAGCAATAAACCAGCCAGCCGGAAGGGC
CGAGCGCAGAAGTGGTCCTGCAACTTTATCCGCCTCCATCCAGTCTATTAAT
TGTTGCCGGGAAGCTAGAGTAAGTAGTTCGCCAGTTAATAGTTTGCGCAAC
GTTGTTGCCATTGCTACAGGCATCGTGGTGTCACGCTCGTCGTTTGGTATGG
CTTCATTCAGCTCCGGTTCCCAACGATCAAGGCGAGTTACATGATCCCCCAT
GTTGTGCAAAAAAGCGGTTAGCTCCTTCGGTCCTCCGATCGTTGTCAGAAG

TAAGTTGGCCGCAGTGTTATCACTCATGGTTATGGCAGCACTGCATAATTCT
CTTACTGTCATGCCATCCGTAAGATGCTTTTCTGTGACTGGTGAGTACTCAA
CCAAGTCATTCTGAGAATAGTGTATGCGGCGACCGAGTTGCTCTTGCCCGG
CGTCAATACGGGATAATACCGCGCCACATAGCAGAACTTTAAAAGTGCTCA
TCATTGGAAAACGTTCTTCGGGGCGAAAACTCTCAAGGATCTTACCGCTGT
TGAGATCCAGTTCGATGTAACCCACTCGTGCACCCAACTGATCTTCAGCAT
CTTTTACTTTCACCAGCGTTTCTGGGTGAGCAAAAACAGGAAGGCAAAAT
GCCGCAAAAAAGGGAATAAGGGCGACACGGAAATGTTGAATACTCATACT
CTTCCTTTTTCAATATTATTGAAGCATTTATCAGGGTTATTGTCTCATGAGCG
GATACATATTTGAATGTATTTAGAAAAATAAACAAATAGGGGTTCCGCGCAC
ATTTCCCCGAAAAGTGCCACCTGACGTCTAAGAAACCATTATTATCATGACA
TTAACCTATAAAAATAGGCGTATCACGAGGCCCTTTCGTCTCGCGCGTTTCG
GTGATGACGGTGAAAACCTCTGACACATGCAGCTCCCGGAGACGGTCACA
GCTTGTCTGTAAGCGGATGCCGGGAGCAGACAAGCCCGTCAGGGCGCGTC
AGCGGGTGTTGGCGGGTGTCGGGGCTGGCTTAACTATGCGGCATCAGAGC
AGATTGTACTGAGAGTGCACCATAAAATTGTAAACGTTAATATTTTGTTAAA
ATTCGCGTTAAATTTTTGTTAAATCAGCTCATTTTTTAACCAATAGGCCGAAA
TCGGCAAAATCCCTTATAAATCAAAAGAATAGCCCGAGATAGGGTTGAGTG
TTGTTCCAGTTTGGAACAAGAGTCCACTATTAAAGAACGTGGACTCCAACG
TCAAAGGGCGAAAAACCGTCTATCAGGGCGATGGCCCACTACGTGAACCA
TCACCCAAATCAAGTTTTTTGGGGTCGAGGTGCCGTAAAGCACTAAATCGG
AACCCTAAAGGGAGCCCCCGATTTAGAGCTTGACGGGGAAAGCCGGCGAA
CGTGGCGAGAAAGGAAGGGAAGAAAGCGAAAGGAGCGGGCGCTAGGGC
GCTGGCAAGTGTAGCGGTCACGCTGCGCGTAACCACCACACCCGCCGCGC
TTAATGCGCCGCTACAGGGCGCGTACTATGGTTGCTTTGACGTATGCGGTGT
GAAATACCGCACAGATGCGTAAGGAGAAAATACCGCATCAGGCGCCCCTG
CAGGCAGCTGCGCGCTCGCTCGCTCACTGAGGCCGCCCGGGCAAAGCCCG
GGCGTCGGGCGACCTTTGGTCGCCCGGCCTCAGTGAGCGAGCGAGCGCGC
AGAGAGGGAGTGGCCAACTCCATCACTAGGGGTTCCTACGCGTCGTTACAT
AACTTACGGTAAATGGCCCGCCTGGCTGACCGCCCAACGACCCCCGCCCAT
TGACGTCAATAATGACGTATGTTCCCATAGTAACGCCAATAGGGACTTTCCA
TTGACGTCAATGGGTGGAGTATTTACGGTAAACTGCCCACTTGGCAGTACA
TCAAGTGTATCATATGCCAAGTACGCCCCCTATTGACGTCAATGACGGTAAA
TGGCCCGCCTGGCATTATGCCCAGTACATGACCTTATGGGACTTTCCTACTT

GGCAGTACATCTACGTATTAGTCATCGCTATTACCATGGTCGAGGTGAGCCC
CACGTTCTGCTTCACTCTCCCCATCTCCCCCCCCTCCCCACCCCCAATTTTG
TATTTATTTATTTTTTAATTATTTTGTGCAGCGATGGGGGCGGGGGGGGGGGG
GGGGCGCGCGCCAGGCGGGGCGGGGCGGGGCGAGGGGCGGGGCGGGG
CGAGGCGGAGAGGTGCGGCGGCAGCCAATCAGAGCGGCGCGCTCCGAAA
GTTTCCTTTTATGGCGAGGCGGCGGCGGCGGCGGCCCTATAAAAAGCGAAG
CGCGCGGCGGGCGGGAGTCGCTGCGACGCTGCCTTCGCCCCGTGCCCCGC
TCCGCCGCCGCCTCGCGCCGCCCGCCCCGGCTCTGACTGACCGCGTTACTC
CCACAGGTGAGCGGGCGGGACGGCCCTTCTCCTCCGGGCTGTAATTAGCTG
AGCAAGAGGTAAGGGTTTAAGGGATGGTTGGTTGGTGGGGTATTAATGTTT
AATTACCTGGAGCACCTGCCTGAAATCACTTTTTTTCAGGTTGGACCGGTG
CCACCATGGCCCTCCTTACGAACCTCCTGCCGTTGTGCTGCCTGGCCCTCCT
CGCCTTGCCAGCGCAGAGCCACGCCGAAGGCACGTTCACCTCCGACGTGA
GCAGCTACCTGGAGGGCCAGGCCGCGAAAGAGTTCATCGCTTGGCTCGTC
AAGGGCGGAGGAGGAGGCGGAGGAAGCGGCGGAGGAGGAAGCGGAGGC
GGAGGTAGCGCCGAGAGCAAGTACGGCCCTCCTTGTCCTCCCTGCCCTGCC
CCAGAGGCCGCTGGAGGACCATCCGTGTTTCTGTTTCCCCCCAAGCCTAAG
GACACCCTGATGATCAGCAGGACCCCCGAGGTGACCTGCGTGGTGGTGGA
CGTGAGCCAGGAGGACCCTGAGGTGCAGTTCAATTGGTACGTGGATGGCG
TGGAGGTGCACAACGCCAAGACAAAGCCCAGAGAGGAGCAGTTTAATAGC
ACCTACAGAGTGGTGTCCGTGCTGACCGTGCTGCACCAGGATTGGCTGAAT
GGCAAGGAGTACAAGTGCAAGGTGTCCAATAAGGGCCTGCCCAGCAGCAT
CGAGAAGACCATCAGCAAGGCCAAGGGCCAGCCCAGAGAGCCTCAGGTG
TACACACTGCCTCCCTCCCAGGAGGAGATGACCAAGAACCAGGTGTCCCT
GACCTGTCTGGTGAAGGGCTTCTACCCCAGCGATATCGCCGTGGAGTGGGA
GTCCAACGGCCAGCCCGAGAATAACTACAAGACCACCCCTCCTGTGCTGGA
TAGCGACGGCAGCTTTTTCCTGTACTCCAGACTGACCGTGGACAAGTCTAG
ATGGCAGGAGGGCAACGTGTTTTCTTGTAGCGTGATGCACGAGGCCCTGCA
CAATCACTACACCCAGAAGTCCCTGTCTCTGAGCCTGGGCTGAGTCGACGT
TTATTTGTGAAATTTGTGATGCTATTGCTTTATTTGTAACCATCTAGCTTTATT
TGTGAAATTTGTGATGCTATTGCTTTATTTGTAACCATTATAAGCTGCAATAA
ACAAGTTAACAACAACAATTGCATTCATTTTATGTTTCAGGTTCAGGGGGA
GATGTGGGAGGTTTTTTAAAGTTTAAACAGGAACCCCTAGTGATGGAGTTG
GCCACTCCCTCTCTGCGCGCTCGCTCGCTCACTGAGGCCGGGCGACCAAA

GGTCGCCCGACGCCCGGGCGGCCTCAGTGAGCGAGCGAGCGCGCAGCTGC
CTGCAGG

Enzyme digestion electrophoresis analysis (Marker: 1kb DNA Ladder) is shown in FIG. 3.

5. The target gene fragment and vector fragment were ligated using Solution I, then transformed into DH5α via heat shock, followed by incubation overnight at 37°C.

### 6. Selection of single colonies for PCR verification.

Single colonies were selected from the transformed plates and inoculated into 400 µL of LB medium containing the corresponding antibiotic, followed by shaking incubation at 37°C and 200 rpm for 2 h.

PCR verification was performed using the following two primers with the bacterial culture after 2-hour shaking incubation as the template and PCR Mix.
Forward primer: ZY005-VL-F (5'→3': GGATAGCAAGGACAGCACATACTC)
Reverse primer: pBR322-R (5'→3': GTGAGCTATGAGAAAGCGCC)

The size of the band after PCR was 736 bp.

PCR electrophoresis analysis (Marker: DL2000 DNA Marker) is shown in FIG. 4.

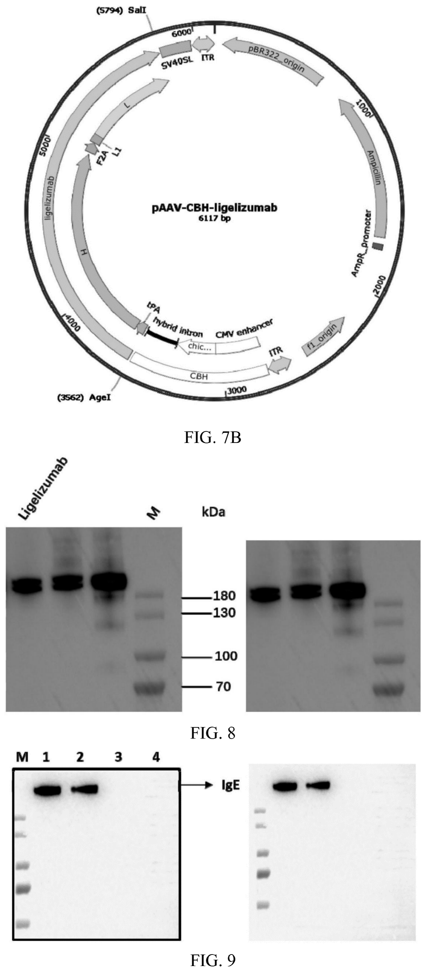

7. The bacterial culture comprising the plasmid with the correct band size was sent for sequencing, and the sequencing results confirmed the successful construction of the plasmid (FIGS. 7A and 7B).

The entire target gene sequence of the above vector was translated as a complete precursor molecule in cells. The self-cleaving 2A sequence and Furin cleavage site were respectively cleaved by enzymes in the cytoplasm to generate highly precise heavy chain and light chain molecules. When the heavy chain and light chain molecules were secreted outside the cell, they assembled into monoclonal antibody molecules with complete structure and function in the cell supernatant.

Furthermore, the present disclosure found that replacing the self-cleaving 2A sequence and Furin cleavage site with IRES may also achieve similar effects.

### Example 2: Packaging and purification of adeno-associated virus expressing Ligelizumab

The antibody gene AAV vector (pAAV-CBH-Ligelizumab) constructed in Example 1, the capsid plasmid AAV8 (with nucleotide sequence shown in SEQ ID NO:25), and the helper plasmid (pHelper, with nucleotide sequence shown in SEQ ID NO:26) were mixed and co-transfected into 293T cells, and AAV vector packaging was performed in this 293T cell line. The transfection method was PEI cationic polymer-mediated transient transfection of eukaryotic cells, and the transfection operation was carried out in accordance with the standardized procedures recommended by the manufacturer, with a transfection scale of 15 cm cell culture dishes. At 7 h post-transfection, the supernatant was aspirated and discarded, and replaced with 25 mL of virus production medium. On day 5 after the completion of transfection, the supernatant and cells were collected and centrifuged at 4200 rpm/min for 10 min. After centrifugation, the supernatant and cells were separated. Lysis buffer and nuclease were added to the cells for lysis and digestion for 1 h, followed by centrifugation at 10000 g/min for 10 min to collect the lysate supernatant. The lysate supernatant and culture medium supernatant were purified by affinity chromatography, then aliquoted and stored frozen at -80°C for later use.

### Example 3: Detection of expression after pAAV-CBH-Ligelizumab infection of cells

The packaged adeno-associated virus was used to infect 293T cells. The culture supernatant of cells after adeno-associated virus infection was subjected to SDS-PAGE electrophoresis, and the expressed antibody was then transferred onto a PVDF membrane. Western blotting assay was performed using HRP-labeled goat anti-human IgG Fc antibody as the secondary antibody. The experimental results (FIG. 8) showed that the adeno-associated virus could effectively express Ligelizumab after transducing cells in vitro and secrete the mature Ligelizumab antibody protein into the cell culture supernatant.

### Example 4: Functional verification of ligelizumab expressed by rAAV

### Western Blot verification of the binding of Ligelizumab to IgE

① The purchased Omalizumab (42.1 µg/µl) was diluted to 1 µg/µL.
② The purchased human IgE protein (1 µg/µl) was diluted to 20 ng/µL.
③ Protein G Agarose was mixed well, and 100 µL was pipetted into three 1.5 mL centrifuge tubes. The resin was washed three times with Binding Buffer (pH 5.0) (by gravity sedimentation, followed by careful aspiration of the supernatant).

Then, IgE, Omalizumab, purified Ligelizumab, and PBS were added according to Table 1 below, and 60 µL of Binding Buffer was added to each for binding at room temperature.

The mixture was gently mixed every 0.5 h, and Protein G Agarose was then allowed to settle by gravity, with a total binding time of 2 h.

**Table 1: Specific data for sample loading**

| | lane 1 | lane 2 | lane 3 | lane 4 |
|---|---|---|---|---|
| Marker | Human IgE 120 ng (6 µL) | Human IgE 120 ng (6 µL) | Human IgE 120 ng (6 µL) | Human IgE 120 ng (6 µL) |
| | | | Omalizumab 2 µg (2 µL) | ligelizumab 2 µg (8 µL) |
| | PBS (54 µL) | Protein G Agarose PBS (54 µL) | Protein G Agarose PBS (52 µL) | Protein G Agarose PBS (46 µL) |
| | | | | |

After 2 h, sample loading was performed with a loading volume of 20 µL (in the case of unbound IgE, this corresponds to 20 ng of IgE added, and the volume of the resin is negligible).
④ Blocking was performed with 5% non-fat milk, followed by three washes.
⑤ Mouse anti-human IgE (Fc) secondary antibody was added for incubation, followed by three washes.
⑥ Exposure was performed.

The experimental results (FIG. 9) indicated that the purified Ligelizumab exhibited the same effect as the purchased Omalizumab, both of which could bind to human IgE, and thus can be used as a standard in subsequent experiments.

### 3. Expression of pAAV-CBH-ligelizumab in mouse myoblast C2C12 cells.

C2C12 cells were infected with the packaged pAAV-CBH-Ligelizumab virus at different MOIs. The virus-infected supernatant was collected, and the content of Ligelizumab in the C2C12 supernatant was determined using purified Ligelizumab as the standard.

The specific procedures were as follows:
A commercial human IgE was used to coat ELISA plates. The expression supernatant and purified Ligelizumab standard served as the primary antibody, and HRP-labeled goat anti-human IgG Fc served as the secondary antibody. TMB was used for color development, and the OD value at 450 nm was measured using a microplate reader. The experimental results (Table 2) showed that the pAAV-CBH-ligelizumab virus could effectively express the anti-human IgE-neutralizing antibody after in vitro transduction of C2C12 cells, and secrete the mature Ligelizumab antibody protein into the cell culture supernatant.

**Table 2: Expression levels of Ligelizumab after infecting cells with different MOIs**

| | | | | |
|---|---|---|---|---|
| MOI | 20000 | 40000 | 80000 | 160000 |
| Ligelizumab (ng/mL) | 21.50 | 19.22 | 52.90 | 94.33 |

### Example 5: In vivo expression of pAAV-CBH-ligelizumab viral vector in normal BALB/c mice

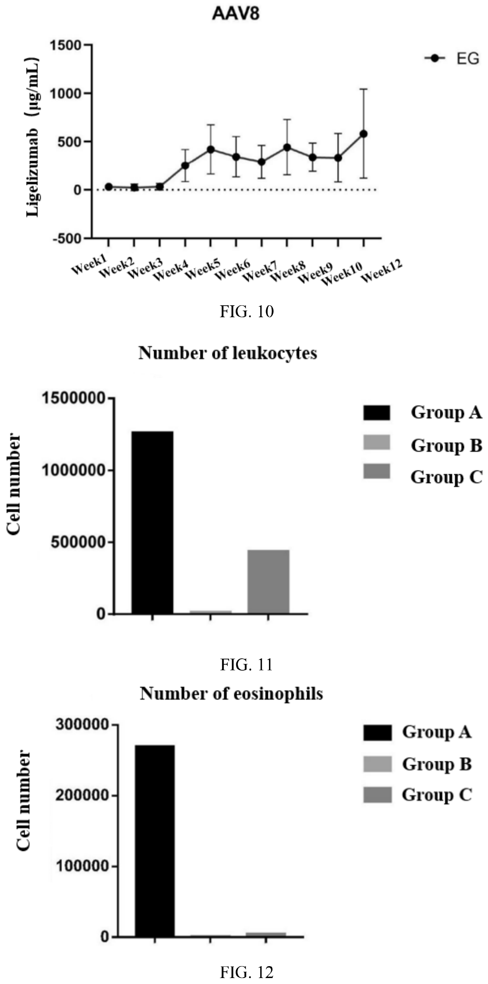

BALB/c mice were administered the pAAV-CBH-Ligelizumab viral vector (experimental group, EG) and normal saline (NG) via intramuscular injection, respectively. Orbital blood was collected from the mice every week to obtain serum. The expression of Ligelizumab in the serum was detected by ELISA. The results (FIG. 10 and Table 3) showed that in the in vivo experiment, the pAAV-CBH-Ligelizumab virus could normally express Ligelizumab in mice, and the expression level was significantly higher than that in the normal saline group.

**Table 3: Ligelizumab antibody concentration in serum of normal BALB/c mice**

| Mous e ID | Ligelizumab (µg/mL) | | | | | | | | | | |
|---|---|---|---|---|---|---|---|---|---|---|---|
| | Wee k 1 | Wee k 2 | Wee k 3 | Wee k 4 | Wee k 5 | Wee k 6 | Wee k 7 | Wee k 8 | Wee k 9 | Week 10 | Week 12 |
| EG-1 | 43.8 | 65.0 | 64.6 | 493. 9 | > Max | 631. 6 | 556. 2 | 970. 4 | 580. 6 | 815.2 | 1398. 0 |
| EG-2 | 32.6 | 11.1 | 37.5 | 309. 5 | 762. 0 | 275. 4 | 207. 0 | 358. 2 | 341. 2 | 223.4 | 491.4 |
| EG-3 | 33.8 | 0.5 | 7.7 | 223. 8 | 478. 6 | 259. 0 | 204. 2 | 279. 6 | 265. 4 | 239.2 | 475.8 |
| EG-4 | 16.2 | 0.2 | 0.5 | 24.9 | 155. 7 | 151. 0 | 157. 7 | 206. 2 | 160. 7 | 89.4 | 36.1 |
| EG-5 | 20.2 | 0.3 | 6.8 | 122. 7 | 176. 8 | 167. 1 | 160. 1 | 271. 6 | 269. 4 | 271.4 | 344.6 |
| EG-6 | 37.4 | 70.6 | 78.6 | 341. 3 | 530. 0 | 580. 6 | 457. 4 | 571. 2 | 420. 2 | 367.4 | 745.8 |
| NC-1 | <Mi n | <Mi n | <Mi n | <Mi n | <Mi n | <Mi n | <Mi n | <Mi n | <Mi n | <Min | <Min |
| NC-2 | <Mi n | <Mi n | <Mi n | <Mi n | <Mi n | <Mi n | <Mi n | <Mi n | <Mi n | <Min | <Min |
| NC-3 | <Mi n | <Mi n | <Mi n | <Mi n | <Mi n | <Mi n | <Mi n | <Mi n | <Mi n | <Min | <Min |
| NC-4 | <Mi n | <Mi n | <Mi n | <Mi n | <Mi n | <Mi n | <Mi n | <Mi n | <Mi n | <Min | <Min |

### Example 6: Therapeutic effect of pAAV-CBH-Ligelizumab viral vector in asthma model animals

The pAAV-CBH-Ligelizumab viral vector was administered to IgE/FcεRI double-humanized BALB/c mice via intramuscular injection 7 days prior to the first model induction (Day-7). At 3 days prior to induction (Day-3), 150 µL of whole blood was collected from the humanized mice for serum separation. Subsequently, after 3 sensitization processes (sensitization by intraperitoneal injection of OVA and aluminum hydroxide gel suspension) and 7 challenge processes (OVA nebulization challenge to induce an asthma model), the mice were sacrificed on Day 28. Serum and bronchoalveolar lavage fluid were collected separately for the determination of IL-4, IL-5, IL-13, OVA-specific IgE, and total IgE. In addition, leukocytes and eosinophils in the bronchoalveolar lavage fluid were counted separately.

The specific protocol is shown in Table 4:

**Table 4: Main research schedule**

| Test drug injection | Serum collection from humanized mice | Mouse sensitization | Nebulization challenge | Collection of serum from all mice and bronchoalveolar lavage fluid from partial mice |
|---|---|---|---|---|
| Day-7 | Day-3 | Day 0, 7, 14 | Day 21 to 27, 30 min per day | Day 28 |

The animals were randomly divided into 3 groups and administered according to Table 5:

**Table 5: Animal grouping and administration**

| Group | Animals | Model inductio n | Ligeli administration | Dosage | Administratio n route and frequency | Number of animals |
|---|---|---|---|---|---|---|
| A | Double-huma nized mice | Yes | No | - | - | 1 |
| B | Double-huma nized mice | No | Yes | H | i.m., once | 1 |
| C | Double-huma nized mice | Yes | Yes | H | i.m., once | 1 |

The experimental results (FIGS. 11 and 12) indicated that the numbers of eosinophils and total leukocytes in the bronchoalveolar lavage fluid of mice were significantly reduced after administration, and the secretion of Th2 cytokines was decreased, with asthma symptoms alleviated.

Based on the above examples, in the present disclosure, the heavy chain and light chain of the human IgE-neutralizing antibody Ligelizumab were fused and expressed via F2A to form a simple and efficient expression framework. The experimental results showed that the pAAV-CBH-Ligelizumab viral vector could be efficiently expressed in cells or mice, and its product could bind to human IgE. The in vivo experiment showed that the pAAV-CBH-Ligelizumab viral vector exhibited an effective blood drug concentration in model mice, and also exhibited positive effects in reducing the number of eosinophils and the secretion of Th2 cytokines in animals.

### Example 7: Construction of pKL-lenti-kan-CBH-ligelizumab vector

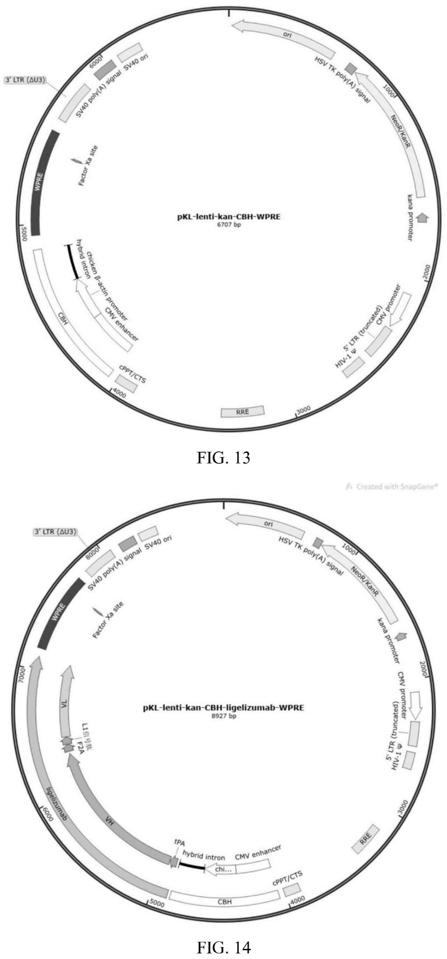

The synthetic gene sequence of the expression cassette for IgE-neutralizing antibody Ligelizumab was amplified by PCR using the plasmid Ligelizumab-pUC57 as the template, and ligated between the MluI and EcoRV of the lentiviral vector pKL-lenti-kan-CBH-WPRE (FIG. 13), which was independently synthesized by Kanglin Biotech, through homologous recombination. After construction, the vector was confirmed to be correct by sequencing and named pKL-lenti-kan-CBH-Ligelizumab (FIG. 14).

### Example 8: Packaging, crude purification, and titer determination of lentiviral pKL-lenti-kan-CBH-Ligelizumab

The Ligelizumab gene lentiviral expression vector pKL-lenti-kan-CBH-Ligelizumab constructed in Example 7, envelope plasmid (Vsvg), and packaging plasmids (Rev and GagPol) were co-transfected into 293T cells (purchased from the American Type Culture Collection (ATCC) with the deposit number CRL-3216), and the lentiviral vector for coagulation factor VIII gene therapy was packaged in this 293T cell line. The method of transfection was PEI cationic polymer-mediated transient transfection of eukaryotic cells. The PEI cationic polymer was PEI-Max transfection reagent purchased from Polysciences, catalog number: 24765-1. The transfection operation was carried out in accordance with the standardized procedures recommended by the manufacturer, with a transfection scale of 10 cm² cell culture dishes.

At 48 hours after the completion of transfection, the lentiviral vector (culture supernatant of transfected cells) was harvested. First, cell debris was removed by centrifugation at 4000 rpm for 5 minutes at room temperature on a benchtop swinging-bucket centrifuge. Then, viral particle precipitates were obtained by centrifugation at 10000g for 4 hours at 4°C. After discarding the centrifuge supernatant, 1 mL of complete DMEM medium was added to the viral particle precipitates, and the viral particles were resuspended using a microsyringe. The prepared viral resuspension was aliquoted and stored frozen at -80°C for later use.

Different volumes of the lentiviral vector were inoculated into the pre-plated human CD4+ T cell line - MT4 cell line (purchased from Shanghai Sure Biotechnology Co., Ltd.) in 96-well cell culture plates. The culture supernatant from cells infected with the EGFP reporter gene lentiviral vector (a lentiviral vector packaged with pCCL-sin-pGK-EGFP according to the foregoing method) was used as a positive control. The infection titer of the initial harvested lentiviral vector supernatant was calculated based on quantitative PCR and flow cytometry data derived from GFP signals by methods well known in the art. The primer and probe sequences used for quantitative PCR were as follows:
LTR-For primer: CTGTTGTGTGACTCTGGTAACT (SEQ ID NO:27)
LTR probe: 5'-AAATCTCTAGCAGTGGCGCCCG-3' (SEQ ID NO:28)
LTR-Rev primer: TTCGCTTTCAAGTCCCTGTT (SEQ ID NO:29)
HK Forward primer 5'-GCTGTCATCTCTTGTGGGCTGT-3' (SEQ ID NO:30)
HK probe 5'-CCTGTCATGCCCACACAAATCTCTCC-3' (SEQ ID NO:31)
HK Reverse primer 5'-ACTCATGGGAGCTGCTGGTTC-3' (SEQ ID NO:32)
Among them, the 5' end of the LTR probe was labeled with a 6FAM fluorophore, and the 3' end with an MGB fluorophore;
the 5' end of the HK probe was labeled with a CY5 fluorophore, and the 3' end with a BHQ2 fluorophore.

The titer of the lentiviral pKL-lenti-kan-CBH-Ligelizumab was determined to be 1.09E+09 TU/mL.

### Example 9: Detection of expression in cells transduced with lentiviral pKL-lenti-kan-CBH-Ligelizumab

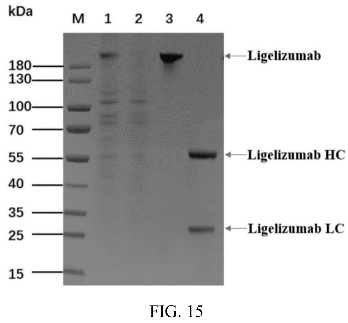

Suspension 293T cells were transduced with lentiviral pKL-lenti-kan-CBH-Ligelizumab at an MOI of 5. After 72 h of culture, the supernatant was collected, and the protein was purified using Protein A resin. After elution, the protein was concentrated, and then buffer-exchanged with PBS to essentially replace the solvent with PBS. Then, the concentration was measured using a BCA kit, adjusted to 250 µg/mL, aliquoted, and stored frozen in a -80°C refrigerator for later use. After purification, SDS-PAGE detection was performed. As shown in FIG. 15, the detection results indicated that the lentiviral vector could express a large amount of Ligelizumab.

### Example 10: Comparison of different 2A peptides in lentiviral vectors

The heavy chain and light chain of the pKL-lenti-kan-CBH-Ligelizumab vector were linked via a Furin cleavage site and the F2A (SEQ ID NO:33) self-cleaving peptide sequence. The effects of T2A (SEQ ID NO:34), E2A (SEQ ID NO:35), and P2A (SEQ ID NO:36) as self-cleaving peptides on Ligelizumab expression were further tested.
F2A: VKQTLNFDLLKLAGDVESNPGP (SEQ ID NO:33)
T2A: EGRGSLL TCGDVEENPGP (SEQ ID NO:34)
E2A: QCTNYALLKLAGDVESNPGP (SEQ ID NO:35)
P2A: ATNFSLLKQAGDVEENPGP (SEQ ID NO:36)

The F2A in pKL-lenti-kan-CBH-Ligelizumab was replaced with T2A, E2A, and P2A, respectively, via a general homologous recombination approach. After construction, the resulting vectors were named pKL-lenti-kan-CBH-Ligelizumab-T2A, pKL-lenti-kan-CBH-Ligelizumab-E2A, and pKL-lenti-kan-CBH-Ligelizumab-P2A, respectively. Lentiviral pKL-lenti-kan-CBH-Ligelizumab, lentiviral pKL-lenti-kan-CBH-Ligelizumab-T2A, pKL-lenti-kan-CBH-Ligelizumab-E2A, and pKL-lenti-kan-CBH-Ligelizumab-P2A were packaged according to the method described in Example 8.

The above four viruses were transduced into 293T cells. (24-well plate, 1.0E+5 cells/well). For each virus, four transduction gradients were set up: 10 µL, 20 µL, 40 µL, 60 µL, 80 µL, and 100 µL. The supernatant and cells were harvested at 72 h. The cells were lysed, and the lentiviral vector copy number (VCN) in infected 293T cells was calculated based on quantitative PCR data by methods well known in the art. The post-transduction supernatants with consistent VCN (about 4) were selected for ELISA detection (see Example 4 for the specific detection method). As shown in Table 6, F2A and T2A exhibited equivalent effects, followed by E2A, with P2A showing the poorest effect.

**Table 6: Comparison of the expression of Ligelizumab expression cassette by different self-cleaving 2A peptides**

| Ligelizumab (ng/mL) | | | |
|---|---|---|---|
| F2A | T2A | E2A | P2A |
| 88.98 | 90.68 | 65.97 | 14.81 |

### Partial Sequence Information

| Seq ID NO: | Name | Sequence |
|---|---|---|
| 1 | Nucleotide sequence of CBH promoter | CGTTACATAACTTACGGTAAATGGCCCGCCTGGCTG ACCGCCCAACGACCCCCGCCCATTGACGTCAATAAT GACGTATGTTCCCATAGTAACGCCAATAGGGACTTT CCATTGACGTCAATGGGTGGAGTATTTACGGTAAAC TGCCCACTTGGCAGTACATCAAGTGTATCATATGCC AAGTACGCCCCCTATTGACGTCAATGACGGTAAATG GCCCGCCTGGCATTATGCCCAGTACATGACCTTATG GGACTTTCCTACTTGGCAGTACATCTACGTATTAGTC ATCGCTATTACCATGGTCGAGGTGAGCCCCACGTTC TGCTTCACTCTCCCCATCTCCCCCCCCTCCCCACCC CCAATTTTGTATTTATTTATTTTTTAATTATTTTGTGC AGCGATGGGGGCGGGGGGGGGGGGGGGGCGCGCG CCAGGCGGGGCGGGGCGGGGCGAGGGGCGGGGCG GGGCGAGGCGGAGAGGTGCGGCGGCAGCCAATCA GAGCGGCGCGCTCCGAAAGTTTCCTTTTATGGCGA GGCGGCGGCGGCGGCGGCCCTATAAAAAGCGAAGC GCGCGGCGGGCGGGAGTCGCTGCGACGCTGCCTTC GCCCCGTGCCCCGCTCCGCCGCCGCCTCGCGCCGC |
| | | CCGCCCCGGCTCTGACTGACCGCGTTACTCCCACA GGTGAGCGGGCGGGACGGCCCTTCTCCTCCGGGCT GTAATTAGCTGAGCAAGAGGTAAGGGTTTAAGGGA TGGTTGGTTGGTGGGGTATTAATGTTTAATTACCTGG AGCACCTGCCTGAAATCACTTTTTTTCAGGT |
| 2 | Nucleotide sequence of heavy chain of Ligelizumab | CAGGTGCAGCTGGTGCAGTCCGGCGCTGAGGTCAT GAAGCCCGGCAGCTCCGTGAAGGTGTCCTGCAAGG CCTCCGGCTACACCTTTAGCTGGTACTGGCTGGAGT GGGTGAGGCAGGCCCCTGGCCACGGACTGGAGTG GATGGGCGAGATCGACCCTGGCACATTCACTACAA ACTACAACGAGAAGTTCAAGGCCAGGGTGACCTTC ACAGCCGATACAAGCACCTCCACAGCCTACATGGA GCTGAGCAGCCTGAGGTCCGAGGACACCGCCGTGT ACTACTGTGCCAGATTTTCCCACTTCAGCGGCTCCA ACTACGACTACTTTGATTACTGGGGCCAGGGCACAC TGGTGACCGTGAGCAGCGCCTCCACCAAGGGCCCT TCCGTGTTCCCTCTGGCCCCTAGCTCCAAGTCCACC TCCGGCGGCACCGCCGCTCTGGGATGTCTGGTGAA GGACTACTTCCCCGAGCCTGTGACCGTGTCCTGGA ACTCCGGCGCCCTGACATCCGGCGTGCACACCTTC CCCGCCGTGCTGCAGAGCTCCGGCCTGTACTCCCT GAGCAGCGTGGTGACAGTGCCTTCCAGCTCCCTGG GCACACAGACCTACATCTGCAACGTGAACCACAAG CCTTCCAATACCAAGGTGGACAAGAAGGTGGAGCC CAAGAGCTGTGATAAGACCCACACCTGTCCCCCTT GCCCTGCCCCCGAGCTGCTGGGAGGACCATCCGTG TTCCTGTTCCCTCCTAAGCCTAAGGACACCCTGATG ATCTCCAGGACCCCCGAGGTGACCTGCGTGGTGGT GGACGTGTCCCACGAGGACCCTGAGGTGAAGTTCA ACTGGTACGTGGATGGCGTGGAGGTGCACAACGCC AAGACAAAGCCTAGGGAGGAGCAGTACAATAGCAC ATACAGAGTGGTGAGCGTGCTGACCGTGCTGCACC AGGACTGGCTGAATGGCAAGGAGTACAAGTGTAAG GTGAGCAATAAGGCCCTGCCCGCCCCCATCGAGAA GACAATCTCCAAGGCCAAGGGCCAGCCTAGAGAGC |
| | | CCCAGGTGTACACACTGCCCCCCTCCAGAGACGAG CTGACAAAGAACCAGGTGTCCCTGACATGTCTGGT GAAAGGCTTTTACCCCAGCGATATCGCCGTGGAGTG GGAGAGCAATGGCCAGCCCGAGAATAATTACAAGA CCACCCCTCCCGTGCTGGACTCCGATGGCAGCTTTT TCCTGTACAGCAAGCTGACCGTGGACAAGAGCAGA TGGCAGCAGGGCAACGTGTTCTCCTGCTCCGTGAT GCACGAGGCCCTGCACAACCACTACACCCAGAAGT CCCTGTCCCTGTCCCCTGGCAAG |
| 3 | Amino acid sequence of heavy chain | QVQLVQSGAEVMKPGSSVKVSCKASGYTFSWYWLE WVRQAPGHGLEWMGEIDPGTFTTNYNEKFKARVTFT ADTSTSTAYMELSSLRSEDTAVYYCARFSHFSGSNYD YFDYWGQGTLVTVSSASTKGPSVFPLAPSSKSTSGGT AALGCLVKDYFPEPVTVSWNSGALTSGVHTFPAVLQS SGLYSLSSVVTVPSSSLGTQTYICNVNHKPSNTKVDK KVEPKSCDKTHTCPPCPAPELLGGPSVFLFPPKPKDTL MISRTPEVTCVVVDVSHEDPEVKFNWYVDGVEVHN AKTKPREEQYNSTYRVVSVLTVLHQDWLNGKEYKC KVSNKALPAPIEKTISKAKGQPREPQVYTLPPSRDELT KNQVSLTCLVKGFYPSDIAVEWESNGQPENNYKTTPP VLDSDGSFFLYSKLTVDKSRWQQGNVFSCSVMHEAL HNHYTQKSLSLSPGK |
| 4 | Nucleotide sequence of light chain of Ligelizumab | GAGATCGTGATGACACAGTCCCCCGCCACCCTGTC CGTGAGCCCAGGAGAGAGGGCCACACTGAGCTGC AGGGCCTCCCAGAGCATCGGCACCAATATCCACTG GTACCAGCAGAAGCCCGGCCAGGCCCCTAGGCTGC TGATCTACTACGCCTCCGAGTCCATCAGCGGCATCC CCGCCAGATTCAGCGGCAGCGGCAGCGGAACCGA GTTTACCCTGACAATCTCCTCCCTGCAGTCCGAGGA CTTCGCCGTGTACTATTGTCAGCAGAGCTGGTCCTG GCCCACCACCTTTGGCGGCGGCACAAAGGTGGAGA TCAAGAGGACAGTGGCCGCCCCTAGCGTGTTTATCT TCCCCCCCAGCGACGAGCAGCTGAAGTCCGGCACC GCCAGCGTGGTGTGTCTGCTGAACAACTTTTACCCT AGGGAGGCCAAGGTGCAGTGGAAGGTGGATAATGC |
| | | CCTGCAGAGCGGCAATTCCCAGGAGTCCGTGACAG AGCAGGATAGCAAGGACAGCACATACTCCCTGTCC AGCACCCTGACACTGTCCAAGGCCGACTACGAGAA GCACAAGGTGTACGCCTGCGAGGTGACACACCAGG GCCTGTCCTCCCCTGTGACAAAGAGCTTCAACAGA GGCGAGTGC |
| 5 | Amino acid sequence of light chain | EIVMTQSPATLSVSPGERATLSCRASQSIGTNIHWYQQ KPGQAPRLLIYYASESISGIPARFSGSGSGTEFTLTISSL QSEDFAVYYCQQSWSWPTTFGGGTKVEIKRTVAAPS VFIFPPSDEQLKSGTASVVCLLNNFYPREAKVQWKVD NALQSGNSQESVTEQDSKDSTYSLSSTLTLSKADYEK HKVYACEVTHQGLSSPVTKSFNRGEC |
| 6 | SV40 polyadenylatio n signal | GTTTATTTGTGAAATTTGTGATGCTATTGCTTTATTTG TAACCATCTAGCTTTATTTGTGAAATTTGTGATGCTA TTGCTTTATTTGTAACCATTATAAGCTGCAATAAACA AGTTAACAACAACAATTGCATTCATTTTATGTTTCAG GTTCAGGGGGAGATGTGGGAGGTTTTTTAAA |
| 7 | Nucleotide sequence of expression cassette | CGTTACATAACTTACGGTAAATGGCCCGCCTGGCTG ACCGCCCAACGACCCCCGCCCATTGACGTCAATAAT GACGTATGTTCCCATAGTAACGCCAATAGGGACTTT CCATTGACGTCAATGGGTGGAGTATTTACGGTAAAC TGCCCACTTGGCAGTACATCAAGTGTATCATATGCC AAGTACGCCCCCTATTGACGTCAATGACGGTAAATG GCCCGCCTGGCATTATGCCCAGTACATGACCTTATG GGACTTTCCTACTTGGCAGTACATCTACGTATTAGTC ATCGCTATTACCATGGTCGAGGTGAGCCCCACGTTC TGCTTCACTCTCCCCATCTCCCCCCCCTCCCCACCC CCAATTTTGTATTTATTTATTTTTTAATTATTTTGTGC AGCGATGGGGGCGGGGGGGGGGGGGGGGCGCGCG CCAGGCGGGGCGGGGCGGGGCGAGGGGCGGGGCG GGGCGAGGCGGAGAGGTGCGGCGGCAGCCAATCA GAGCGGCGCGCTCCGAAAGTTTCCTTTTATGGCGA GGCGGCGGCGGCGGCGGCCCTATAAAAAGCGAAGC GCGCGGCGGGCGGGAGTCGCTGCGACGCTGCCTTC GCCCCGTGCCCCGCTCCGCCGCCGCCTCGCGCCGC |
| | | CCGCCCCGGCTCTGACTGACCGCGTTACTCCCACA GGTGAGCGGGCGGGACGGCCCTTCTCCTCCGGGCT GTAATTAGCTGAGCAAGAGGTAAGGGTTTAAGGGA TGGTTGGTTGGTGGGGTATTAATGTTTAATTACCTGG AGCACCTGCCTGAAATCACTTTTTTTCAGGTTGGAC CGGTGCCACCATGGACGCCATGAAGAGGGGCCTGT GTTGTGTGCTGCTGCTGTGCGGCGCCGTGTTTGTGA GCCCCCAGGTGCAGCTGGTGCAGTCCGGCGCTGAG GTCATGAAGCCCGGCAGCTCCGTGAAGGTGTCCTG CAAGGCCTCCGGCTACACCTTTAGCTGGTACTGGCT GGAGTGGGTGAGGCAGGCCCCTGGCCACGGACTG GAGTGGATGGGCGAGATCGACCCTGGCACATTCAC TACAAACTACAACGAGAAGTTCAAGGCCAGGGTGA CCTTCACAGCCGATACAAGCACCTCCACAGCCTACA TGGAGCTGAGCAGCCTGAGGTCCGAGGACACCGCC GTGTACTACTGTGCCAGATTTTCCCACTTCAGCGGC TCCAACTACGACTACTTTGATTACTGGGGCCAGGGC ACACTGGTGACCGTGAGCAGCGCCTCCACCAAGGG CCCTTCCGTGTTCCCTCTGGCCCCTAGCTCCAAGTC CACCTCCGGCGGCACCGCCGCTCTGGGATGTCTGG TGAAGGACTACTTCCCCGAGCCTGTGACCGTGTCCT GGAACTCCGGCGCCCTGACATCCGGCGTGCACACC TTCCCCGCCGTGCTGCAGAGCTCCGGCCTGTACTCC CTGAGCAGCGTGGTGACAGTGCCTTCCAGCTCCCT GGGCACACAGACCTACATCTGCAACGTGAACCACA AGCCTTCCAATACCAAGGTGGACAAGAAGGTGGAG CCCAAGAGCTGTGATAAGACCCACACCTGTCCCCC TTGCCCTGCCCCCGAGCTGCTGGGAGGACCATCCG TGTTCCTGTTCCCTCCTAAGCCTAAGGACACCCTGA TGATCTCCAGGACCCCCGAGGTGACCTGCGTGGTG GTGGACGTGTCCCACGAGGACCCTGAGGTGAAGTT CAACTGGTACGTGGATGGCGTGGAGGTGCACAACG CCAAGACAAAGCCTAGGGAGGAGCAGTACAATAGC ACATACAGAGTGGTGAGCGTGCTGACCGTGCTGCA CCAGGACTGGCTGAATGGCAAGGAGTACAAGTGTA |
| | | AGGTGAGCAATAAGGCCCTGCCCGCCCCCATCGAG AAGACAATCTCCAAGGCCAAGGGCCAGCCTAGAGA GCCCCAGGTGTACACACTGCCCCCCTCCAGAGACG AGCTGACAAAGAACCAGGTGTCCCTGACATGTCTG GTGAAAGGCTTTTACCCCAGCGATATCGCCGTGGAG TGGGAGAGCAATGGCCAGCCCGAGAATAATTACAA GACCACCCCTCCCGTGCTGGACTCCGATGGCAGCT TTTTCCTGTACAGCAAGCTGACCGTGGACAAGAGC AGATGGCAGCAGGGCAACGTGTTCTCCTGCTCCGT GATGCACGAGGCCCTGCACAACCACTACACCCAGA AGTCCCTGTCCCTGTCCCCTGGCAAGAGGAAGAGA AGGGCCCCCGTGAAGCAGACCCTGAACTTCGATCT GCTGAAGCTGGCCGGCGATGTGGAGTCCAATCCCG GCCCTATGGACATGAGGGTGCCCGCCCAGCTGCTG GGCCTGCTGCTGCTGTGGCTGAGCGGCGCTAGATG CGAGATCGTGATGACACAGTCCCCCGCCACCCTGT CCGTGAGCCCAGGAGAGAGGGCCACACTGAGCTG CAGGGCCTCCCAGAGCATCGGCACCAATATCCACT GGTACCAGCAGAAGCCCGGCCAGGCCCCTAGGCTG CTGATCTACTACGCCTCCGAGTCCATCAGCGGCATC CCCGCCAGATTCAGCGGCAGCGGCAGCGGAACCGA GTTTACCCTGACAATCTCCTCCCTGCAGTCCGAGGA CTTCGCCGTGTACTATTGTCAGCAGAGCTGGTCCTG GCCCACCACCTTTGGCGGCGGCACAAAGGTGGAGA TCAAGAGGACAGTGGCCGCCCCTAGCGTGTTTATCT TCCCCCCCAGCGACGAGCAGCTGAAGTCCGGCACC GCCAGCGTGGTGTGTCTGCTGAACAACTTTTACCCT AGGGAGGCCAAGGTGCAGTGGAAGGTGGATAATGC CCTGCAGAGCGGCAATTCCCAGGAGTCCGTGACAG AGCAGGATAGCAAGGACAGCACATACTCCCTGTCC AGCACCCTGACACTGTCCAAGGCCGACTACGAGAA GCACAAGGTGTACGCCTGCGAGGTGACACACCAGG GCCTGTCCTCCCCTGTGACAAAGAGCTTCAACAGA GGCGAGTGCTAGGTCGACGTTTATTTGTGAAATTTG TGATGCTATTGCTTTATTTGTAACCATCTAGCTTTATT |
| | | TGTGAAATTTGTGATGCTATTGCTTTATTTGTAACCA TTATAAGCTGCAATAAACAAGTTAACAACAACAATT GCATTCATTTTATGTTTCAGGTTCAGGGGGAGATGT GGGAGGTTTTTTAAA |
| 8 | nucleotide sequence of L-ITR | CCTGCAGGCAGCTGCGCGCTCGCTCGCTCACTGAG GCCGCCCGGGCAAAGCCCGGGCGTCGGGCGACCTT TGGTCGCCCGGCCTCAGTGAGCGAGCGAGCGCGCA GAGAGGGAGTGGCCAACTCCATCACTAGGGGTTCC T |
| 9 | nucleotide sequence of R-ITR | AGGAACCCCTAGTGATGGAGTTGGCCACTCCCTCT CTGCGCGCTCGCTCGCTCACTGAGGCCGGGCGACC AAAGGTCGCCCGACGCCCGGGCTTTGCCCGGGCGG CCTCAGTGAGCGAGCGAGCGCGCAGCTGCCTGCAG G |
| 10 | Sequence of adeno-associat ed virus backbone vector | ACATGTGAGCAAAAGGCCAGCAAAAGGCCAGGAA CCGTAAAAAGGCCGCGTTGCTGGCGTTTTTCCATAG GCTCCGCCCCCCTGACGAGCATCACAAAAATCGAC GCTCAAGTCAGAGGTGGCGAAACCCGACAGGACTA TAAAGATACCAGGCGTTTCCCCCTGGAAGCTCCCTC GTGCGCTCTCCTGTTCCGACCCTGCCGCTTACCGGA TACCTGTCCGCCTTTCTCCCTTCGGGAAGCGTGGCG CTTTCTCATAGCTCACGCTGTAGGTATCTCAGTTCG GTGTAGGTCGTTCGCTCCAAGCTGGGCTGTGTGCA CGAACCCCCCGTTCAGCCCGACCGCTGCGCCTTATC CGGTAACTATCGTCTTGAGTCCAACCCGGTAAGACA CGACTTATCGCCACTGGCAGCAGCCACTGGTAACA GGATTAGCAGAGCGAGGTATGTAGGCGGTGCTACA GAGTTCTTGAAGTGGTGGCCTAACTACGGCTACACT AGAAGGACAGTATTTGGTATCTGCGCTCTGCTGAAG CCAGTTACCTTCGGAAAAAGAGTTGGTAGCTCTTGA TCCGGCAAACAAACCACCGCTGGTAGCGGTGGTTT TTTTGTTTGCAAGCAGCAGATTACGCGCAGAAAAA AAGGATCTCAAGAAGATCCTTTGATCTTTTCTACGG GGTCTGACGCTCAGTGGAACGAAAACTCACGTTAA GGGATTTTGGTCATGAGATTATCAAAAAGGATCTTC |
| | | ACCTAGATCCTTTTAAATTAAAAATGAAGTTTTAAAT CAATCTAAAGTATATATGAGTAAACTTGGTCTGACA GTTACCAATGCTTAATCAGTGAGGCACCTATCTCAG CGATCTGTCTATTTCGTTCATCCATAGTTGCCTGACT CCCCGTCGTGTAGATAACTACGATACGGGAGGGCTT ACCATCTGGCCCCAGTGCTGCAATGATACCGCGAG ACCCACGCTCACCGGCTCCAGATTTATCAGCAATAA ACCAGCCAGCCGGAAGGGCCGAGCGCAGAAGTGG TCCTGCAACTTTATCCGCCTCCATCCAGTCTATTAAT TGTTGCCGGGAAGCTAGAGTAAGTAGTTCGCCAGTT AATAGTTTGCGCAACGTTGTTGCCATTGCTACAGGC ATCGTGGTGTCACGCTCGTCGTTTGGTATGGCTTCA TTCAGCTCCGGTTCCCAACGATCAAGGCGAGTTACA TGATCCCCCATGTTGTGCAAAAAAGCGGTTAGCTCC TTCGGTCCTCCGATCGTTGTCAGAAGTAAGTTGGCC GCAGTGTTATCACTCATGGTTATGGCAGCACTGCAT AATTCTCTTACTGTCATGCCATCCGTAAGATGCTTTT CTGTGACTGGTGAGTACTCAACCAAGTCATTCTGAG AATAGTGTATGCGGCGACCGAGTTGCTCTTGCCCGG CGTCAATACGGGATAATACCGCGCCACATAGCAGAA CTTTAAAAGTGCTCATCATTGGAAAACGTTCTTCGG GGCGAAAACTCTCAAGGATCTTACCGCTGTTGAGAT CCAGTTCGATGTAACCCACTCGTGCACCCAACTGAT CTTCAGCATCTTTTACTTTCACCAGCGTTTCTGGGT GAGCAAAAACAGGAAGGCAAAATGCCGCAAAAAA GGGAATAAGGGCGACACGGAAATGTTGAATACTCA TACTCTTCCTTTTTCAATATTATTGAAGCATTTATCAG GGTTATTGTCTCATGAGCGGATACATATTTGAATGTA TTTAGAAAAATAAACAAATAGGGGTTCCGCGCACAT TTCCCCGAAAAGTGCCACCTGACGTCTAAGAAACC ATTATTATCATGACATTAACCTATAAAAATAGGCGTA TCACGAGGCCCTTTCGTCTCGCGCGTTTCGGTGATG ACGGTGAAAACCTCTGACACATGCAGCTCCCGGAG ACGGTCACAGCTTGTCTGTAAGCGGATGCCGGGAG CAGACAAGCCCGTCAGGGCGCGTCAGCGGGTGTTG |
| | | GCGGGTGTCGGGGCTGGCTTAACTATGCGGCATCA GAGCAGATTGTACTGAGAGTGCACCATAAAATTGTA AACGTTAATATTTTGTTAAAATTCGCGTTAAATTTTT GTTAAATCAGCTCATTTTTTAACCAATAGGCCGAAA TCGGCAAAATCCCTTATAAATCAAAAGAATAGCCCG AGATAGGGTTGAGTGTTGTTCCAGTTTGGAACAAG AGTCCACTATTAAAGAACGTGGACTCCAACGTCAA AGGGCGAAAAACCGTCTATCAGGGCGATGGCCCAC TACGTGAACCATCACCCAAATCAAGTTTTTTGGGGT CGAGGTGCCGTAAAGCACTAAATCGGAACCCTAAA GGGAGCCCCCGATTTAGAGCTTGACGGGGAAAGCC GGCGAACGTGGCGAGAAAGGAAGGGAAGAAAGCG AAAGGAGCGGGCGCTAGGGCGCTGGCAAGTGTAG CGGTCACGCTGCGCGTAACCACCACACCCGCCGCG CTTAATGCGCCGCTACAGGGCGCGTACTATGGTTGC TTTGACGTATGCGGTGTGAAATACCGCACAGATGCG TAAGGAGAAAATACCGCATCAGGCGCCCCTGCAGG CAGCTGCGCGCTCGCTCGCTCACTGAGGCCGCCCG GGCAAAGCCCGGGCGTCGGGCGACCTTTGGTCGCC CGGCCTCAGTGAGCGAGCGAGCGCGCAGAGAGGG AGTGGCCAACTCCATCACTAGGGGTTCCTACGCGTC GTTACATAACTTACGGTAAATGGCCCGCCTGGCTGA CCGCCCAACGACCCCCGCCCATTGACGTCAATAATG ACGTATGTTCCCATAGTAACGCCAATAGGGACTTTC CATTGACGTCAATGGGTGGAGTATTTACGGTAAACT GCCCACTTGGCAGTACATCAAGTGTATCATATGCCA AGTACGCCCCCTATTGACGTCAATGACGGTAAATGG CCCGCCTGGCATTATGCCCAGTACATGACCTTATGG GACTTTCCTACTTGGCAGTACATCTACGTATTAGTCA TCGCTATTACCATGGTCGAGGTGAGCCCCACGTTCT GCTTCACTCTCCCCATCTCCCCCCCCTCCCCACCCC CAATTTTGTATTTATTTATTTTTTAATTATTTTGTGCA GCGATGGGGGCGGGGGGGGGGGGGGGGCGCGCGC CAGGCGGGGCGGGGCGGGGCGAGGGGCGGGGCGG GGCGAGGCGGAGAGGTGCGGCGGCAGCCAATCAG |
| | | AGCGGCGCGCTCCGAAAGTTTCCTTTTATGGCGAG GCGGCGGCGGCGGCGGCCCTATAAAAAGCGAAGCG CGCGGCGGGCGGGAGTCGCTGCGACGCTGCCTTCG CCCCGTGCCCCGCTCCGCCGCCGCCTCGCGCCGCC CGCCCCGGCTCTGACTGACCGCGTTACTCCCACAG GTGAGCGGGCGGGACGGCCCTTCTCCTCCGGGCTG TAATTAGCTGAGCAAGAGGTAAGGGTTTAAGGGAT GGTTGGTTGGTGGGGTATTAATGTTTAATTACCTGG AGCACCTGCCTGAAATCACTTTTTTTCAGGTTGGAC CGGTGCCACCGGTCGACGTTTATTTGTGAAATTTGT GATGCTATTGCTTTATTTGTAACCATCTAGCTTTATTT GTGAAATTTGTGATGCTATTGCTTTATTTGTAACCAT TATAAGCTGCAATAAACAAGTTAACAACAACAATTG CATTCATTTTATGTTTCAGGTTCAGGGGGAGATGTG GGAGGTTTTTTAAAGTTTAAACAGGAACCCCTAGT GATGGAGTTGGCCACTCCCTCTCTGCGCGCTCGCTC GCTCACTGAGGCCGGGCGACCAAAGGTCGCCCGAC GCCCGGGCGGCCTCAGTGAGCGAGCGAGCGCGCA GCTGCCTGCAGG |
| 11 | Sequence of adeno-associated virus vector comprising target gene | ACATGTGAGCAAAAGGCCAGCAAAAGGCCAGGAA CCGTAAAAAGGCCGCGTTGCTGGCGTTTTTCCATAG GCTCCGCCCCCCTGACGAGCATCACAAAAATCGAC GCTCAAGTCAGAGGTGGCGAAACCCGACAGGACTA TAAAGATACCAGGCGTTTCCCCCTGGAAGCTCCCTC GTGCGCTCTCCTGTTCCGACCCTGCCGCTTACCGGA TACCTGTCCGCCTTTCTCCCTTCGGGAAGCGTGGCG CTTTCTCATAGCTCACGCTGTAGGTATCTCAGTTCG GTGTAGGTCGTTCGCTCCAAGCTGGGCTGTGTGCA CGAACCCCCCGTTCAGCCCGACCGCTGCGCCTTATC CGGTAACTATCGTCTTGAGTCCAACCCGGTAAGACA CGACTTATCGCCACTGGCAGCAGCCACTGGTAACA GGATTAGCAGAGCGAGGTATGTAGGCGGTGCTACA GAGTTCTTGAAGTGGTGGCCTAACTACGGCTACACT AGAAGGACAGTATTTGGTATCTGCGCTCTGCTGAAG CCAGTTACCTTCGGAAAAAGAGTTGGTAGCTCTTGA |
| | | TCCGGCAAACAAACCACCGCTGGTAGCGGTGGTTT TTTTGTTTGCAAGCAGCAGATTACGCGCAGAAAAA AAGGATCTCAAGAAGATCCTTTGATCTTTTCTACGG GGTCTGACGCTCAGTGGAACGAAAACTCACGTTAA GGGATTTTGGTCATGAGATTATCAAAAAGGATCTTC ACCTAGATCCTTTTAAATTAAAAATGAAGTTTTAAAT CAATCTAAAGTATATATGAGTAAACTTGGTCTGACA GTTACCAATGCTTAATCAGTGAGGCACCTATCTCAG CGATCTGTCTATTTCGTTCATCCATAGTTGCCTGACT CCCCGTCGTGTAGATAACTACGATACGGGAGGGCTT ACCATCTGGCCCCAGTGCTGCAATGATACCGCGAG ACCCACGCTCACCGGCTCCAGATTTATCAGCAATAA ACCAGCCAGCCGGAAGGGCCGAGCGCAGAAGTGG TCCTGCAACTTTATCCGCCTCCATCCAGTCTATTAAT TGTTGCCGGGAAGCTAGAGTAAGTAGTTCGCCAGTT AATAGTTTGCGCAACGTTGTTGCCATTGCTACAGGC ATCGTGGTGTCACGCTCGTCGTTTGGTATGGCTTCA TTCAGCTCCGGTTCCCAACGATCAAGGCGAGTTACA TGATCCCCCATGTTGTGCAAAAAAGCGGTTAGCTCC TTCGGTCCTCCGATCGTTGTCAGAAGTAAGTTGGCC GCAGTGTTATCACTCATGGTTATGGCAGCACTGCAT AATTCTCTTACTGTCATGCCATCCGTAAGATGCTTTT CTGTGACTGGTGAGTACTCAACCAAGTCATTCTGAG AATAGTGTATGCGGCGACCGAGTTGCTCTTGCCCGG CGTCAATACGGGATAATACCGCGCCACATAGCAGAA CTTTAAAAGTGCTCATCATTGGAAAACGTTCTTCGG GGCGAAAACTCTCAAGGATCTTACCGCTGTTGAGAT CCAGTTCGATGTAACCCACTCGTGCACCCAACTGAT CTTCAGCATCTTTTACTTTCACCAGCGTTTCTGGGT GAGCAAAAACAGGAAGGCAAAATGCCGCAAAAAA GGGAATAAGGGCGACACGGAAATGTTGAATACTCA TACTCTTCCTTTTTCAATATTATTGAAGCATTTATCAG GGTTATTGTCTCATGAGCGGATACATATTTGAATGTA TTTAGAAAAATAAACAAATAGGGGTTCCGCGCACAT TTCCCCGAAAAGTGCCACCTGACGTCTAAGAAACC |
| | | ATTATTATCATGACATTAACCTATAAAAATAGGCGTA TCACGAGGCCCTTTCGTCTCGCGCGTTTCGGTGATG ACGGTGAAAACCTCTGACACATGCAGCTCCCGGAG ACGGTCACAGCTTGTCTGTAAGCGGATGCCGGGAG CAGACAAGCCCGTCAGGGCGCGTCAGCGGGTGTTG GCGGGTGTCGGGGCTGGCTTAACTATGCGGCATCA GAGCAGATTGTACTGAGAGTGCACCATAAAATTGTA AACGTTAATATTTTGTTAAAATTCGCGTTAAATTTTT GTTAAATCAGCTCATTTTTTAACCAATAGGCCGAAA TCGGCAAAATCCCTTATAAATCAAAAGAATAGCCCG AGATAGGGTTGAGTGTTGTTCCAGTTTGGAACAAG AGTCCACTATTAAAGAACGTGGACTCCAACGTCAA AGGGCGAAAAACCGTCTATCAGGGCGATGGCCCAC TACGTGAACCATCACCCAAATCAAGTTTTTTGGGGT CGAGGTGCCGTAAAGCACTAAATCGGAACCCTAAA GGGAGCCCCCGATTTAGAGCTTGACGGGGAAAGCC GGCGAACGTGGCGAGAAAGGAAGGGAAGAAAGCG AAAGGAGCGGGCGCTAGGGCGCTGGCAAGTGTAG CGGTCACGCTGCGCGTAACCACCACACCCGCCGCG CTTAATGCGCCGCTACAGGGCGCGTACTATGGTTGC TTTGACGTATGCGGTGTGAAATACCGCACAGATGCG TAAGGAGAAAATACCGCATCAGGCGCCCCTGCAGG CAGCTGCGCGCTCGCTCGCTCACTGAGGCCGCCCG GGCAAAGCCCGGGCGTCGGGCGACCTTTGGTCGCC CGGCCTCAGTGAGCGAGCGAGCGCGCAGAGAGGG AGTGGCCAACTCCATCACTAGGGGTTCCTACGCGTC GTTACATAACTTACGGTAAATGGCCCGCCTGGCTGA CCGCCCAACGACCCCCGCCCATTGACGTCAATAATG ACGTATGTTCCCATAGTAACGCCAATAGGGACTTTC CATTGACGTCAATGGGTGGAGTATTTACGGTAAACT GCCCACTTGGCAGTACATCAAGTGTATCATATGCCA AGTACGCCCCCTATTGACGTCAATGACGGTAAATGG CCCGCCTGGCATTATGCCCAGTACATGACCTTATGG GACTTTCCTACTTGGCAGTACATCTACGTATTAGTCA TCGCTATTACCATGGTCGAGGTGAGCCCCACGTTCT |
| | | GCTTCACTCTCCCCATCTCCCCCCCCTCCCCACCCC CAATTTTGTATTTATTTATTTTTTAATTATTTTGTGCA GCGATGGGGGCGGGGGGGGGGGGGGGGCGCGCGC CAGGCGGGGCGGGGCGGGGCGAGGGGCGGGGCGG GGCGAGGCGGAGAGGTGCGGCGGCAGCCAATCAG AGCGGCGCGCTCCGAAAGTTTCCTTTTATGGCGAG GCGGCGGCGGCGGCGGCCCTATAAAAAGCGAAGCG CGCGGCGGGCGGGAGTCGCTGCGACGCTGCCTTCG CCCCGTGCCCCGCTCCGCCGCCGCCTCGCGCCGCC CGCCCCGGCTCTGACTGACCGCGTTACTCCCACAG GTGAGCGGGCGGGACGGCCCTTCTCCTCCGGGCTG TAATTAGCTGAGCAAGAGGTAAGGGTTTAAGGGAT GGTTGGTTGGTGGGGTATTAATGTTTAATTACCTGG AGCACCTGCCTGAAATCACTTTTTTTCAGGTTGGAC CGGTGCCACCATGGACGCCATGAAGAGGGGCCTGT GTTGTGTGCTGCTGCTGTGCGGCGCCGTGTTTGTGA GCCCCCAGGTGCAGCTGGTGCAGTCCGGCGCTGAG GTCATGAAGCCCGGCAGCTCCGTGAAGGTGTCCTG CAAGGCCTCCGGCTACACCTTTAGCTGGTACTGGCT GGAGTGGGTGAGGCAGGCCCCTGGCCACGGACTG GAGTGGATGGGCGAGATCGACCCTGGCACATTCAC TACAAACTACAACGAGAAGTTCAAGGCCAGGGTGA CCTTCACAGCCGATACAAGCACCTCCACAGCCTACA TGGAGCTGAGCAGCCTGAGGTCCGAGGACACCGCC GTGTACTACTGTGCCAGATTTTCCCACTTCAGCGGC TCCAACTACGACTACTTTGATTACTGGGGCCAGGGC ACACTGGTGACCGTGAGCAGCGCCTCCACCAAGGG CCCTTCCGTGTTCCCTCTGGCCCCTAGCTCCAAGTC CACCTCCGGCGGCACCGCCGCTCTGGGATGTCTGG TGAAGGACTACTTCCCCGAGCCTGTGACCGTGTCCT GGAACTCCGGCGCCCTGACATCCGGCGTGCACACC TTCCCCGCCGTGCTGCAGAGCTCCGGCCTGTACTCC CTGAGCAGCGTGGTGACAGTGCCTTCCAGCTCCCT GGGCACACAGACCTACATCTGCAACGTGAACCACA AGCCTTCCAATACCAAGGTGGACAAGAAGGTGGAG |
| | | CCCAAGAGCTGTGATAAGACCCACACCTGTCCCCC TTGCCCTGCCCCCGAGCTGCTGGGAGGACCATCCG TGTTCCTGTTCCCTCCTAAGCCTAAGGACACCCTGA TGATCTCCAGGACCCCCGAGGTGACCTGCGTGGTG GTGGACGTGTCCCACGAGGACCCTGAGGTGAAGTT CAACTGGTACGTGGATGGCGTGGAGGTGCACAACG CCAAGACAAAGCCTAGGGAGGAGCAGTACAATAGC ACATACAGAGTGGTGAGCGTGCTGACCGTGCTGCA CCAGGACTGGCTGAATGGCAAGGAGTACAAGTGTA AGGTGAGCAATAAGGCCCTGCCCGCCCCCATCGAG AAGACAATCTCCAAGGCCAAGGGCCAGCCTAGAGA GCCCCAGGTGTACACACTGCCCCCCTCCAGAGACG AGCTGACAAAGAACCAGGTGTCCCTGACATGTCTG GTGAAAGGCTTTTACCCCAGCGATATCGCCGTGGAG TGGGAGAGCAATGGCCAGCCCGAGAATAATTACAA GACCACCCCTCCCGTGCTGGACTCCGATGGCAGCT TTTTCCTGTACAGCAAGCTGACCGTGGACAAGAGC AGATGGCAGCAGGGCAACGTGTTCTCCTGCTCCGT GATGCACGAGGCCCTGCACAACCACTACACCCAGA AGTCCCTGTCCCTGTCCCCTGGCAAGAGGAAGAGA AGGGCCCCCGTGAAGCAGACCCTGAACTTCGATCT GCTGAAGCTGGCCGGCGATGTGGAGTCCAATCCCG GCCCTATGGACATGAGGGTGCCCGCCCAGCTGCTG GGCCTGCTGCTGCTGTGGCTGAGCGGCGCTAGATG CGAGATCGTGATGACACAGTCCCCCGCCACCCTGT CCGTGAGCCCAGGAGAGAGGGCCACACTGAGCTG CAGGGCCTCCCAGAGCATCGGCACCAATATCCACT GGTACCAGCAGAAGCCCGGCCAGGCCCCTAGGCTG CTGATCTACTACGCCTCCGAGTCCATCAGCGGCATC CCCGCCAGATTCAGCGGCAGCGGCAGCGGAACCGA GTTTACCCTGACAATCTCCTCCCTGCAGTCCGAGGA CTTCGCCGTGTACTATTGTCAGCAGAGCTGGTCCTG GCCCACCACCTTTGGCGGCGGCACAAAGGTGGAGA TCAAGAGGACAGTGGCCGCCCCTAGCGTGTTTATCT TCCCCCCCAGCGACGAGCAGCTGAAGTCCGGCACC |
| | | GCCAGCGTGGTGTGTCTGCTGAACAACTTTTACCCT AGGGAGGCCAAGGTGCAGTGGAAGGTGGATAATGC CCTGCAGAGCGGCAATTCCCAGGAGTCCGTGACAG AGCAGGATAGCAAGGACAGCACATACTCCCTGTCC AGCACCCTGACACTGTCCAAGGCCGACTACGAGAA GCACAAGGTGTACGCCTGCGAGGTGACACACCAGG GCCTGTCCTCCCCTGTGACAAAGAGCTTCAACAGA GGCGAGTGCTAGGTCGACGTTTATTTGTGAAATTTG TGATGCTATTGCTTTATTTGTAACCATCTAGCTTTATT TGTGAAATTTGTGATGCTATTGCTTTATTTGTAACCA TTATAAGCTGCAATAAACAAGTTAACAACAACAATT GCATTCATTTTATGTTTCAGGTTCAGGGGGAGATGT GGGAGGTTTTTTAAAGTTTAAACAGGAACCCCTAG TGATGGAGTTGGCCACTCCCTCTCTGCGCGCTCGCT CGCTCACTGAGGCCGGGCGACCAAAGGTCGCCCGA CGCCCGGGCTTTGCCCGGGCGGCCTCAGTGAGCGA GCGAGCGCGCAGCTGCCTGCAGG |
| 12 | Nucleotide sequence of TMCK promoter | TCTAAGCTAGACCCTTCAGATTAAAAATAACTGAGG TAAGGGCCTGGGTAGGGGAGGTGGTGTGAGACGCT CCTGTCTCTCCTCTATCTGCCCATCGGCCCTTTGGG GAGGAGGAATGTGCCCAAGGACTAAAAAAAGGCC ATGGAGCCAGAGGGGCGAGGGCAACAGACCTTTCA TGGGCAAACCTTGGGGCCCTGCTGTCTAGCATGCC CCACTACGGGTCTAGGCTGCCCATGTAAGGAGGCA AGGCCTGGGGACACCCGAGATGCCTGGTTATAATTA ACCCAGACATGTGGCTGCCCCCCCCCCCCCAACAC CTGCTGCCTCTAAAAATAACCCTGTCCCTGGTGGAT CCCCTGCATGCGAAGATCTTCGAACAAGGCTGTGG GGGACTGAGGGCAGGCTGTAACAGGCTTGGGGGCC AGGGCTTATACGTGCCTGGGACTCCCAAAGTATTAC TGTTCCATGTTCCCGGCGAAGGGCCAGCTGTCCCCC GCCAGCTAGACTCAGCACTTAGTTTAGGAACCAGT GAGCAAGTCAGCCCTTGGGGCAGCCCATACAAGGC CATGGGGCTGGGCAAGCTGCACGCCTGGGTCCGGG GTGGGCACGGTGCCCGGGCAACGAGCTGAAAGCT |
| | | CATCTGCTCTCAGGGGCCCCTCCCTGGGGACAGCC CCTCCTGGCTAGTCACACCCTGTAGGCTCCTCTATAT AACCCAGG |
| 13 | Nucleotide sequence of MCK promoter | CAGCCACTATGGGTCTAGGCTGCCCATGTAAGGAG GCAAGGCCTGGGGACACCCGAGATGCCTGGTTATA ATTAACCCAGACATGTGGCTGCTCCCCCCCCCCAAC ACCTGCTGCCTGAGCCTCACCCCCACCCCGGTGCC TGGGTCTTAGGCTCTGTACACCATGGAGGAGAAGC TCGCTCTAAAAATAACCCTGTCCCTGGTGGGCTGTG GGGGACTGAGGGCAGGCTGTAACAGGCTTGGGGG CCAGGGCTTATACGTGCCTGGGACTCCCAAAGTATT ACTGTTCCATGTTCCCGGCGAAGGGCCAGCTGTCC CCCGCCAGCTAGACTCAGCACTTAGTTTAGGAACC AGTGAGCAAGTCAGCCCTTGGGGCAGCCCATACAA GGCCATGGGGCTGGGCAAGCTGCACGCCTGGGTCC GGGGTGGGCACGGTGCCCGGGCAACGAGCTGAAA GCTCATCTGCTCTCAGGGGCCCCTCCCTGGGGACA GCCCCTCCTGGCTAGTCACACCCTGTAGGCTCCTCT ATATAACCCAGGGGCACAGGGGCTGCCCCCGGGTC AC |
| 14 | Nucleotide sequence of MHCK7 promoter | GTTTAAACAAGCTTGCATGTCTAAGCTAGACCCTTC AGATTAAAAATAACTGAGGTAAGGGCCTGGGTAGG GGAGGTGGTGTGAGACGCTCCTGTCTCTCCTCTATC TGCCCATCGGCCCTTTGGGGAGGAGGAATGTGCCC AAGGACTAAAAAAAGGCCATGGAGCCAGAGGGGC GAGGGCAACAGACCTTTCATGGGCAAACCTTGGGG CCCTGCTGTCTAGCATGCCCCACTACGGGTCTAGGC TGCCCATGTAAGGAGGCAAGGCCTGGGGACACCCG AGATGCCTGGTTATAATTAACCCAGACATGTGGCTG CCCCCCCCCCCCCAACACCTGCTGCCTCTAAAAATA ACCCTGTCCCTGGTGGATCCCCTGCATGCGAAGATC TTCGAACAAGGCTGTGGGGGACTGAGGGCAGGCT GTAACAGGCTTGGGGGCCAGGGCTTATACGTGCCT GGGACTCCCAAAGTATTACTGTTCCATGTTCCCGGC |
| | | GAAGGGCCAGCTGTCCCCCGCCAGCTAGACTCAGC ACTTAGTTTAGGAACCAGTGAGCAAGTCAGCCCTT GGGGCAGCCCATACAAGGCCATGGGGCTGGGCAAG CTGCACGCCTGGGTCCGGGGTGGGCACGGTGCCCG GGCAACGAGCTGAAAGCTCATCTGCTCTCAGGGGC CCCTCCCTGGGGACAGCCCCTCCTGGCTAGTCACA CCCTGTAGGCTCCTCTATATAACCCAGGGGCACAGG GGCTGCCCTCATTCTACCACCACCTCCACAGCACAG ACAGACACTCAGGAGCCAGCCAGCGGCGCGCCC |
| 15 | Nucleotide sequence of TTR promoter | GGGGGAGGCTGCTGGTGAATATTAACCAAGGTCAC CCCAGTTATCGGAGGAGCAAACAGGGGCTAAGTCC ACTCTTGCATCTAAAATGAGAGACAAAAAATCTATA AAAATGGAAAACATGCATAGAAATATGTGAGGGAG GAAAAAATTACCCCCAAGAATGTTAGTGCACGCAG TCACACAGGGAGAAGACTATTTTTGTTTTGTTTTGA TTGTTTTGTTTTGTTTTGGTTGTTTTGTTTTGGTGAC CTAACTGGTCAAATGACCTATTAAGAATATTTCATAG AACGAATGTTCCGATGCTCTAATCTCTCTAGACAAG GTTCATATTTGTATGGGTTACTTATTCTCTCTTTGTTG ACTAAGTCAATAATCAGAATCAGCAGGTTTGCAGTC AGATTGGCAGGGATAAGCAGCCTAGCTCAGGAGAA GTGAGTATAAAAGCCCCAGGCTGGGAGCAGCCATC ACAGAAGTCCA |
| 16 | Amino acid sequence of SAP signal peptide | MKWVTFISLLFLFSSAYS |
| 17 | Amino acid sequence of IHC signal peptide | MDWTWRVFCLLAVTPGAHP |
| 18 | Amino acid sequence of ILC signal peptide | MAWSPLFLTLITHCAGSWA |
| 19 | Amino acid sequence of AP signal peptide | MTRLTVLALLAGLLASSRA |
| 20 | Amino acid sequence of CSP signal peptide | MARPLCTLLLLMATLAGALA |
| 21 | Amino acid sequence of IL2 signal peptide | MYRMQLLSCIALSLALVTNS |
| 22 | Amino acid sequence of TPA signal peptide | MDAMKRGLCCVLLLCGAVFVSP |
| 23 | Amino acid sequence of L1 signal peptide | MDMRVPAQLLGLLLLWLSGARC |
| 24 | Amino acid sequence of H7 signal peptide | MEFGLSWVFLVALFRGVQC |
| 25 | Nucleotide sequence of packaging plasmid AAV8 | ATGCCGGGGTTTTACGAGATTGTGATTAAGGTCCCC AGCGACCTTGACGAGCATCTGCCCGGCATTTCTGAC AGCTTTGTGAACTGGGTGGCCGAGAAGGAATGGGA GTTGCCGCCAGATTCTGACATGGATCTGAATCTGAT TGAGCAGGCACCCCTGACCGTGGCCGAGAAGCTGC AGCGCGACTTTCTGACGGAATGGCGCCGTGTGAGT AAGGCCCCGGAGGCTCTTTTCTTTGTGCAATTTGAG AAGGGAGAGAGCTACTTCCACATGCACGTGCTCGT GGAAACCACCGGGGTGAAATCCATGGTTTTGGGAC |
| | | GTTTCCTGAGTCAGATTCGCGAAAAACTGATTCAG AGAATTTACCGCGGGATCGAGCCGACTTTGCCAAA CTGGTTCGCGGTCACAAAGACCAGAAATGGCGCCG GAGGCGGGAACAAGGTGGTGGATGAGTGCTACATC CCCAATTACTTGCTCCCCAAAACCCAGCCTGAGCTC CAGTGGGCGTGGACTAATATGGAACAGTATTTAAGC GCCTGTTTGAATCTCACGGAGCGTAAACGGTTGGT GGCGCAGCATCTGACGCACGTGTCGCAGACGCAGG AGCAGAACAAAGAGAATCAGAATCCCAATTCTGAT GCGCCGGTGATCAGATCAAAAACTTCAGCCAGGTA CATGGAGCTGGTCGGGTGGCTCGTGGACAAGGGGA TTACCTCGGAGAAGCAGTGGATCCAGGAGGACCAG GCCTCATACATCTCCTTCAATGCGGCCTCCAACTCG CGGTCCCAAATCAAGGCTGCCTTGGACAATGCGGG AAAGATTATGAGCCTGACTAAAACCGCCCCCGACT ACCTGGTGGGCCAGCAGCCCGTGGAGGACATTTCC AGCAATCGGATTTATAAAATTTTGGAACTAAACGGG TACGATCCCCAATATGCGGCTTCCGTCTTTCTGGGAT GGGCCACGAAAAAGTTCGGCAAGAGGAACACCAT CTGGCTGTTTGGGCCTGCAACTACCGGGAAGACCA ACATCGCGGAGGCCATAGCCCACACTGTGCCCTTCT ACGGGTGCGTAAACTGGACCAATGAGAACTTTCCC TTCAACGACTGTGTCGACAAGATGGTGATCTGGTG GGAGGAGGGGAAGATGACCGCCAAGGTCGTGGAG TCGGCCAAAGCCATTCTCGGAGGAAGCAAGGTGCG CGTGGACCAGAAATGCAAGTCCTCGGCCCAGATAG ACCCGACTCCCGTGATCGTCACCTCCAACACCAAC ATGTGCGCCGTGATTGACGGGAACTCAACGACCTT CGAACACCAGCAGCCGTTGCAAGACCGGATGTTCA AATTTGAACTCACCCGCCGTCTGGATCATGACTTTG GGAAGGTCACCAAGCAGGAAGTCAAAGACTTTTTC CGGTGGGCAAAGGATCACGTGGTTGAGGTGGAGCA TGAATTCTACGTCAAAAAGGGTGGAGCCAAGAAAA GACCCGCCCCCAGTGACGCAGATATAAGTGAGCCC AAACGGGTGCGCGAGTCAGTTGCGCAGCCATCGAC |
| | | GTCAGACGCGGAAGCTTCGATCAACTACGCAGACA GGTACCAAAACAAATGTTCTCGTCACGTGGGCATG AATCTGATGCTGTTTCCCTGCAGACAATGCGAGAGA ATGAATCAGAATTCAAATATCTGCTTCACTCACGGA CAGAAAGACTGTTTAGAGTGCTTTCCCGTGTCAGA ATCTCAACCCGTTTCTGTCGTCAAAAAGGCGTATCA GAAACTGTGCTACATTCATCATATCATGGGAAAGGT GCCAGACGCTTGCACTGCCTGCGATCTGGTCAATGT GGATTTGGATGACTGCATCTTTGAACAATAAATGAT TTAAATCAGGTATGGCTGCCGATGGTTATCTTCCAGA TTGGCTCGAGGACAACCTCTCTGAGGGCATTCGCG AGTGGTGGGCGCTGAAACCTGGAGCCCCGAAGCCC AAAGCCAACCAGCAAAAGCAGGACGACGGCCGGG GTCTGGTGCTTCCTGGCTACAAGTACCTCGGACCCT TCAACGGACTCGACAAGGGGGAGCCCGTCAACGC GGCGGACGCAGCGGCCCTCGAGCACGACAAGGCC TACGACCAGCAGCTGCAGGCGGGTGACAATCCGTA CCTGCGGTATAACCACGCCGACGCCGAGTTTCAGG AGCGTCTGCAAGAAGATACGTCTTTTGGGGGCAAC CTCGGGCGAGCAGTCTTCCAGGCCAAGAAGCGGGT TCTCGAACCTCTCGGTCTGGTTGAGGAAGGCGCTA AGACGGCTCCTGGAAAGAAGAGACCGGTAGAGCC ATCACCCCAGCGTTCTCCAGACTCCTCTACGGGCAT CGGCAAGAAAGGCCAACAGCCCGCCAGAAAAAGA CTCAATTTTGGTCAGACTGGCGACTCAGAGTCAGTT CCAGACCCTCAACCTCTCGGAGAACCTCCAGCAGC GCCCTCTGGTGTGGGACCTAATACAATGGCTGCAGG CGGTGGCGCACCAATGGCAGACAATAACGAAGGCG CCGACGGAGTGGGTAGTTCCTCGGGAAATTGGCAT TGCGATTCCACATGGCTGGGCGACAGAGTCATCAC CACCAGCACCCGAACCTGGGCCCTGCCCACCTACA ACAACCACCTCTACAAGCAAATCTCCAACGGGACA TCGGGAGGAGCCACCAACGACAACACCTACTTCGG CTACAGCACCCCCTGGGGGTATTTTGACTTTAACAG ATTCCACTGCCACTTTTCACCACGTGACTGGCAGCG |
| | | ACTCATCAACAACAACTGGGGATTCCGGCCCAAGA GACTCAGCTTCAAGCTCTTCAACATCCAGGTCAAG GAGGTCACGCAGAATGAAGGCACCAAGACCATCGC CAATAACCTCACCAGCACCATCCAGGTGTTTACGGA CTCGGAGTACCAGCTGCCGTACGTTCTCGGCTCTGC CCACCAGGGCTGCCTGCCTCCGTTCCCGGCGGACG TGTTCATGATTCCCCAGTACGGCTACCTAACACTCA ACAACGGTAGTCAGGCCGTGGGACGCTCCTCCTTC TACTGCCTGGAATACTTTCCTTCGCAGATGCTGAGA ACCGGCAACAACTTCCAGTTTACTTACACCTTCGAG GACGTGCCTTTCCACAGCAGCTACGCCCACAGCCA GAGCTTGGACCGGCTGATGAATCCTCTGATTGACCA GTACCTGTACTACTTGTCTCGGACTCAAACAACAGG AGGCACGGCAAATACGCAGACTCTGGGCTTCAGCC AAGGTGGGCCTAATACAATGGCCAATCAGGCAAAG AACTGGCTGCCAGGACCCTGTTACCGCCAACAACG CGTCTCAACGACAACCGGGCAAAACAACAATAGCA ACTTTGCCTGGACTGCTGGGACCAAATACCATCTGA ATGGAAGAAATTCATTGGCTAATCCTGGCATCGCTA TGGCAACACACAAAGACGACGAGGAGCGTTTTTTT CCCAGTAACGGGATCCTGATTTTTGGCAAACAAAAT GCTGCCAGAGACAATGCGGATTACAGCGATGTCATG CTCACCAGCGAGGAAGAAATCAAAACCACTAACCC TGTGGCTACAGAGGAATACGGTATCGTGGCAGATAA CTTGCAGCAGCAAAACACGGCTCCTCAAATTGGAA CTGTCAACAGCCAGGGGGCCTTACCCGGTATGGTCT GGCAGAACCGGGACGTGTACCTGCAGGGTCCCATC TGGGCCAAGATTCCTCACACGGACGGCAACTTCCA CCCGTCTCCGCTGATGGGCGGCTTTGGCCTGAAACA TCCTCCGCCTCAGATCCTGATCAAGAACACGCCTGT ACCTGCGGATCCTCCGACCACCTTCAACCAGTCAA AGCTGAACTCTTTCATCACGCAATACAGCACCGGA CAGGTCAGCGTGGAAATTGAATGGGAGCTGCAGAA GGAAAACAGCAAGCGCTGGAACCCCGAGATCCAGT ACACCTCCAACTACTACAAATCTACAAGTGTGGACT |
| | | TTGCTGTTAATACAGAAGGCGTGTACTCTGAACCCC GCCCCATTGGCACCCGTTACCTCACCCGTAATCTGT AATTGCCTGTTAATCAATAAACCGGTTGATTCGTTTC AGTTGAACTTTGGTCTCTGCGAAGGGCGAATTCGTT TAAACCTGCAGGACTAGAGGTCCTGTATTAGAGGTC ACGTGAGTGTTTTGCGACATTTTGCGACACCATGTG GTCACGCTGGGTATTTAAGCCCGAGTGAGCACGCA GGGTCTCCATTTTGAAGCGGGAGGTTTGAACGCGC AGCCGCCAAGCCGAATTCTGCAGATATCCATCACAC TGGCGGCCGCTCGACTAGAGCGGCCGCCACCGCGG TGGAGCTCCAGCTTTTGTTCCCTTTAGTGAGGGTTA ATTGCGCGCTTGGCGTAATCATGGTCATAGCTGTTTC CTGTGTGAAATTGTTATCCGCTCACAATTCCACACA ACATACGAGCCGGAAGCATAAAGTGTAAAGCCTGG GGTGCCTAATGAGTGAGCTAACTCACATTAATTGCG TTGCGCTCACTGCCCGCTTTCCAGTCGGGAAACCT GTCGTGCCAGCTGCATTAATGAATCGGCCAACGCGC GGGGAGAGGCGGTTTGCGTATTGGGCGCTCTTCCG CTTCCTCGCTCACTGACTCGCTGCGCTCGGTCGTTC GGCTGCGGCGAGCGGTATCAGCTCACTCAAAGGCG GTAATACGGTTATCCACAGAATCAGGGGATAACGCA GGAAAGAACATGTGAGCAAAAGGCCAGCAAAAGG CCAGGAACCGTAAAAAGGCCGCGTTGCTGGCGTTT TTCCATAGGCTCCGCCCCCCTGACGAGCATCACAAA AATCGACGCTCAAGTCAGAGGTGGCGAAACCCGAC AGGACTATAAAGATACCAGGCGTTTCCCCCTGGAA GCTCCCTCGTGCGCTCTCCTGTTCCGACCCTGCCGC TTACCGGATACCTGTCCGCCTTTCTCCCTTCGGGAA GCGTGGCGCTTTCTCATAGCTCACGCTGTAGGTATC TCAGTTCGGTGTAGGTCGTTCGCTCCAAGCTGGGCT GTGTGCACGAACCCCCCGTTCAGCCCGACCGCTGC GCCTTATCCGGTAACTATCGTCTTGAGTCCAACCCG GTAAGACACGACTTATCGCCACTGGCAGCAGCCAC TGGTAACAGGATTAGCAGAGCGAGGTATGTAGGCG GTGCTACAGAGTTCTTGAAGTGGTGGCCTAACTAC |
| | | GGCTACACTAGAAGAACAGTATTTGGTATCTGCGCT CTGCTGAAGCCAGTTACCTTCGGAAAAAGAGTTGG TAGCTCTTGATCCGGCAAACAAACCACCGCTGGTA GCGGTGGTTTTTTTGTTTGCAAGCAGCAGATTACGC GCAGAAAAAAAGGATCTCAAGAAGATCCTTTGATC TTTTCTACGGGGTCTGACGCTCAGTGGAACGAAAA CTCACGTTAAGGGATTTTGGTCATGAGATTATCAAA AAGGATCTTCACCTAGATCCTTTTAAATTAAAAATG AAGTTTTAAATCAATCTAAAGTATATATGAGTAAACT TGGTCTGACAGTTACCAATGCTTAATCAGTGAGGCA CCTATCTCAGCGATCTGTCTATTTCGTTCATCCATAG TTGCCTGACTCCCCGTCGTGTAGATAACTACGATAC GGGAGGGCTTACCATCTGGCCCCAGTGCTGCAATG ATACCGCGAGACCCACGCTCACCGGCTCCAGATTTA TCAGCAATAAACCAGCCAGCCGGAAGGGCCGAGC GCAGAAGTGGTCCTGCAACTTTATCCGCCTCCATCC AGTCTATTAATTGTTGCCGGGAAGCTAGAGTAAGTA GTTCGCCAGTTAATAGTTTGCGCAACGTTGTTGCCA TTGCTACAGGCATCGTGGTGTCACGCTCGTCGTTTG GTATGGCTTCATTCAGCTCCGGTTCCCAACGATCAA GGCGAGTTACATGATCCCCCATGTTGTGCAAAAAAG CGGTTAGCTCCTTCGGTCCTCCGATCGTTGTCAGAA GTAAGTTGGCCGCAGTGTTATCACTCATGGTTATGG CAGCACTGCATAATTCTCTTACTGTCATGCCATCCGT AAGATGCTTTTCTGTGACTGGTGAGTACTCAACCAA GTCATTCTGAGAATAGTGTATGCGGCGACCGAGTTG CTCTTGCCCGGCGTCAATACGGGATAATACCGCGCC ACATAGCAGAACTTTAAAAGTGCTCATCATTGGAAA ACGTTCTTCGGGGCGAAAACTCTCAAGGATCTTACC GCTGTTGAGATCCAGTTCGATGTAACCCACTCGTGC ACCCAACTGATCTTCAGCATCTTTTACTTTCACCAG CGTTTCTGGGTGAGCAAAAACAGGAAGGCAAAATG CCGCAAAAAAGGGAATAAGGGCGACACGGAAATG TTGAATACTCATACTCTTCCTTTTTCAATATTATTGAA GCATTTATCAGGGTTATTGTCTCATGAGCGGATACAT |
| | | ATTTGAATGTATTTAGAAAAATAAACAAATAGGGGT TCCGCGCACATTTCCCCGAAAAGTGCCACCTAAATT GTAAGCGTTAATATTTTGTTAAAATTCGCGTTAAATT TTTGTTAAATCAGCTCATTTTTTAACCAATAGGCCGA AATCGGCAAAATCCCTTATAAATCAAAAGAATAGAC CGAGATAGGGTTGAGTGTTGTTCCAGTTTGGAACA AGAGTCCACTATTAAAGAACGTGGACTCCAACGTC AAAGGGCGAAAAACCGTCTATCAGGGCGATGGCCC ACTACGTGAACCATCACCCTAATCAAGTTTTTTGGG GTCGAGGTGCCGTAAAGCACTAAATCGGAACCCTA AAGGGAGCCCCCGATTTAGAGCTTGACGGGGAAAG CCGGCGAACGTGGCGAGAAAGGAAGGGAAGAAAG CGAAAGGAGCGGGCGCTAGGGCGCTGGCAAGTGT AGCGGTCACGCTGCGCGTAACCACCACACCCGCCG CGCTTAATGCGCCGCTACAGGGCGCGTCCCATTCGC CATTCAGGCTGCGCAACTGTTGGGAAGGGCGATCG GTGCGGGCCTCTTCGCTATTACGCCAGCTGGCGAAA GGGGGATGTGCTGCAAGGCGATTAAGTTGGGTAAC GCCAGGGTTTTCCCAGTCACGACGTTGTAAAACGA CGGCCAGTGAGCGCGCGTAATACGACTCACTATAGG GCGAATTGGGTACCGGGCCCCCCCTCGATCGAGGT CGACGGTATCGGGGGAGCTCGCAGGGTCTCCATTTT GAAGCGGGAGGTTTGAACGCGCAGCCGCC |
| 26 | Nucleotide sequence of helper plasmid pHelper | GGTACCCAACTCCATGCTTAACAGTCCCCAGGTACA GCCCACCCTGCGTCGCAACCAGGAACAGCTCTACA GCTTCCTGGAGCGCCACTCGCCCTACTTCCGCAGCC ACAGTGCGCAGATTAGGAGCGCCACTTCTTTTTGTC ACTTGAAAAACATGTAAAAATAATGTACTAGGAGAC ACTTTCAATAAAGGCAAATGTTTTTATTTGTACACTC TCGGGTGATTATTTACCCCCCACCCTTGCCGTCTGC GCCGTTTAAAAATCAAAGGGGTTCTGCCGCGCATC GCTATGCGCCACTGGCAGGGACACGTTGCGATACT GGTGTTTAGTGCTCCACTTAAACTCAGGCACAACCA TCCGCGGCAGCTCGGTGAAGTTTTCACTCCACAGG CTGCGCACCATCACCAACGCGTTTAGCAGGTCGGG |
| | | CGCCGATATCTTGAAGTCGCAGTTGGGGCCTCCGCC CTGCGCGCGCGAGTTGCGATACACAGGGTTGCAGC ACTGGAACACTATCAGCGCCGGGTGGTGCACGCTG GCCAGCACGCTCTTGTCGGAGATCAGATCCGCGTC CAGGTCCTCCGCGTTGCTCAGGGCGAACGGAGTCA ACTTTGGTAGCTGCCTTCCCAAAAAGGGTGCATGCC CAGGCTTTGAGTTGCACTCGCACCGTAGTGGCATCA GAAGGTGACCGTGCCCGGTCTGGGCGTTAGGATAC AGCGCCTGCATGAAAGCCTTGATCTGCTTAAAAGCC ACCTGAGCCTTTGCGCCTTCAGAGAAGAACATGCC GCAAGACTTGCCGGAAAACTGATTGGCCGGACAGG CCGCGTCATGCACGCAGCACCTTGCGTCGGTGTTG GAGATCTGCACCACATTTCGGCCCCACCGGTTCTTC ACGATCTTGGCCTTGCTAGACTGCTCCTTCAGCGCG CGCTGCCCGTTTTCGCTCGTCACATCCATTTCAATC ACGTGCTCCTTATTTATCATAATGCTCCCGTGTAGAC ACTTAAGCTCGCCTTCGATCTCAGCGCAGCGGTGC AGCCACAACGCGCAGCCCGTGGGCTCGTGGTGCTT GTAGGTTACCTCTGCAAACGACTGCAGGTACGCCT GCAGGAATCGCCCCATCATCGTCACAAAGGTCTTGT TGCTGGTGAAGGTCAGCTGCAACCCGCGGTGCTCC TCGTTTAGCCAGGTCTTGCATACGGCCGCCAGAGCT TCCACTTGGTCAGGCAGTAGCTTGAAGTTTGCCTTT AGATCGTTATCCACGTGGTACTTGTCCATCAACGCG CGCGCAGCCTCCATGCCCTTCTCCCACGCAGACAC GATCGGCAGGCTCAGCGGGTTTATCACCGTGCTTTC ACTTTCCGCTTCACTGGACTCTTCCTTTTCCTCTTGC GTCCGCATACCCCGCGCCACTGGGTCGTCTTCATTC AGCCGCCGCACCGTGCGCTTACCTCCCTTGCCGTGC TTGATTAGCACCGGTGGGTTGCTGAAACCCACCATT TGTAGCGCCACATCTTCTCTTTCTTCCTCGCTGTCCA CGATCACCTCTGGGGATGGCGGGCGCTCGGGCTTG GGAGAGGGGCGCTTCTTTTTCTTTTTGGACGCAATG GCCAAATCCGCCGTCGAGGTCGATGGCCGCGGGCT GGGTGTGCGCGGCACCAGCGCATCTTGTGACGAGT |
| | | CTTCTTCGTCCTCGGACTCGAGACGCCGCCTCAGCC GCTTTTTTGGGGGCGCGCGGGGAGGCGGCGGCGAC GGCGACGGGGACGACACGTCCTCCATGGTTGGTGG ACGTCGCGCCGCACCGCGTCCGCGCTCGGGGGTGG TTTCGCGCTGCTCCTCTTCCCGACTGGCCATTTCCTT CTCCTATAGGCAGAAAAAGATCATGGAGTCAGTCG AGAAGGAGGACAGCCTAACCGCCCCCTTTGAGTTC GCCACCACCGCCTCCACCGATGCCGCCAACGCGCC TACCACCTTCCCCGTCGAGGCACCCCCGCTTGAGG AGGAGGAAGTGATTATCGAGCAGGACCCAGGTTTT GTAAGCGAAGACGACGAGGATCGCTCAGTACCAAC AGAGGATAAAAAGCAAGACCAGGACGACGCAGAG GCAAACGAGGAACAAGTCGGGCGGGGGGACCAAA GGCATGGCGACTACCTAGATGTGGGAGACGACGTG CTGTTGAAGCATCTGCAGCGCCAGTGCGCCATTATC TGCGACGCGTTGCAAGAGCGCAGCGATGTGCCCCT CGCCATAGCGGATGTCAGCCTTGCCTACGAACGCCA CCTGTTCTCACCGCGCGTACCCCCCAAACGCCAAG AAAACGGCACATGCGAGCCCAACCCGCGCCTCAAC TTCTACCCCGTATTTGCCGTGCCAGAGGTGCTTGCC ACCTATCACATCTTTTTCCAAAACTGCAAGATACCC CTATCCTGCCGTGCCAACCGCAGCCGAGCGGACAA GCAGCTGGCCTTGCGGCAGGGCGCTGTCATACCTG ATATCGCCTCGCTCGACGAAGTGCCAAAAATCTTTG AGGGTCTTGGACGCGACGAGAAACGCGCGGCAAA CGCTCTGCAACAAGAAAACAGCGAAAATGAAAGT CACTGTGGAGTGCTGGTGGAACTTGAGGGTGACAA CGCGCGCCTAGCCGTGCTGAAACGCAGCATCGAGG TCACCCACTTTGCCTACCCGGCACTTAACCTACCCC CCAAGGTTATGAGCACAGTCATGAGCGAGCTGATC GTGCGCCGTGCACGACCCCTGGAGAGGGATGCAAA CTTGCAAGAACAAACCGAGGAGGGCCTACCCGCA GTTGGCGATGAGCAGCTGGCGCGCTGGCTTGAGAC GCGCGAGCCTGCCGACTTGGAGGAGCGACGCAAG CTAATGATGGCCGCAGTGCTTGTTACCGTGGAGCTT |
| | | GAGTGCATGCAGCGGTTCTTTGCTGACCCGGAGAT GCAGCGCAAGCTAGAGGAAACGTTGCACTACACCT TTCGCCAGGGCTACGTGCGCCAGGCCTGCAAAATT TCCAACGTGGAGCTCTGCAACCTGGTCTCCTACCTT GGAATTTTGCACGAAAACCGCCTCGGGCAAAACGT GCTTCATTCCACGCTCAAGGGCGAGGCGCGCCGCG ACTACGTCCGCGACTGCGTTTACTTATTTCTGTGCTA CACCTGGCAAACGGCCATGGGCGTGTGGCAGCAAT GCCTGGAGGAGCGCAACCTAAAGGAGCTGCAGAA GCTGCTAAAGCAAAACTTGAAGGACCTATGGACGG CCTTCAACGAGCGCTCCGTGGCCGCGCACCTGGCG GACATTATCTTCCCCGAACGCCTGCTTAAAACCCTG CAACAGGGTCTGCCAGACTTCACCAGTCAAAGCAT GTTGCAAAACTTTAGGAACTTTATCCTAGAGCGTTC AGGAATTCTGCCCGCCACCTGCTGTGCGCTTCCTAG CGACTTTGTGCCCATTAAGTACCGTGAATGCCCTCC GCCGCTTTGGGGTCACTGCTACCTTCTGCAGCTAGC CAACTACCTTGCCTACCACTCCGACATCATGGAAGA CGTGAGCGGTGACGGCCTACTGGAGTGTCACTGTC GCTGCAACCTATGCACCCCGCACCGCTCCCTGGTCT GCAATTCGCAACTGCTTAGCGAAAGTCAAATTATCG GTACCTTTGAGCTGCAGGGTCCCTCGCCTGACGAA AAGTCCGCGGCTCCGGGGTTGAAACTCACTCCGGG GCTGTGGACGTCGGCTTACCTTCGCAAATTTGTACC TGAGGACTACCACGCCCACGAGATTAGGTTCTACG AAGACCAATCCCGCCCGCCAAATGCGGAGCTTACC GCCTGCGTCATTACCCAGGGCCACATCCTTGGCCAA TTGCAAGCCATCAACAAAGCCCGCCAAGAGTTTCT GCTACGAAAGGGACGGGGGGTTTACCTGGACCCCC AGTCCGGCGAGGAGCTCAACCCAATCCCCCCGCCG CCGCAGCCCTATCAGCAGCCGCGGGCCCTTGCTTCC CAGGATGGCACCCAAAAAGAAGCTGCAGCTGCCGC CGCCGCCACCCACGGACGAGGAGGAATACTGGGAC AGTCAGGCAGAGGAGGTTTTGGACGAGGAGGAGG AGATGATGGAAGACTGGGACAGCCTAGACGAAGCT |
| | | TCCGAGGCCGAAGAGGTGTCAGACGAAACACCGT CACCCTCGGTCGCATTCCCCTCGCCGGCGCCCCAG AAATTGGCAACCGTTCCCAGCATCGCTACAACCTCC GCTCCTCAGGCGCCGCCGGCACTGCCTGTTCGCCG ACCCAACCGTAGATGGGACACCACTGGAACCAGGG CCGGTAAGTCTAAGCAGCCGCCGCCGTTAGCCCAA GAGCAACAACAGCGCCAAGGCTACCGCTCGTGGCG CGGGCACAAGAACGCCATAGTTGCTTGCTTGCAAG ACTGTGGGGGCAACATCTCCTTCGCCCGCCGCTTTC TTCTCTACCATCACGGCGTGGCCTTCCCCCGTAACA TCCTGCATTACTACCGTCATCTCTACAGCCCCTACTG CACCGGCGGCAGCGGCAGCGGCAGCAACAGCAGC GGTCACACAGAAGCAAAGGCGACCGGATAGCAAG ACTCTGACAAAGCCCAAGAAATCCACAGCGGCGGC AGCAGCAGGAGGAGGAGCGCTGCGTCTGGCGCCC AACGAACCCGTATCGACCCGCGAGCTTAGAAATAG GATTTTTCCCACTCTGTATGCTATATTTCAACAAAGC AGGGGCCAAGAACAAGAGCTGAAAATAAAAAACA GGTCTCTGCGCTCCCTCACCCGCAGCTGCCTGTATC ACAAAAGCGAAGATCAGCTTCGGCGCACGCTGGAA GACGCGGAGGCTCTCTTCAGCAAATACTGCGCGCT GACTCTTAAGGACTAGTTTCGCGCCCTTTCTCAAAT TTAAGCGCGAAAACTACGTCATCTCCAGCGGCCAC ACCCGGCGCCAGCACCTGTCGTCAGCGCCATTATG AGCAAGGAAATTCCCACGCCCTACATGTGGAGTTA CCAGCCACAAATGGGACTTGCGGCTGGAGCTGCCC AAGACTACTCAACCCGAATAAACTACATGAGCGCG GGACCCCACATGATATCCCGGGTCAACGGAATCCGC GCCCACCGAAACCGAATTCTCCTCGAACAGGCGGC TATTACCACCACACCTCGTAATAACCTTAATCCCCGT AGTTGGCCCGCTGCCCTGGTGTACCAGGAAAGTCC CGCTCCCACCACTGTGGTACTTCCCAGAGACGCCC AGGCCGAAGTTCAGATGACTAACTCAGGGGCGCAG CTTGCGGGCGGCTTTCGTCACAGGGTGCGGTCGCC CGGGCGTTTTAGGGCGGAGTAACTTGCATGTATTGG |
| | | GAATTGTAGTTTTTTTAAAATGGGAAGTGACGTATC GTGGGAAAACGGAAGTGAAGATTTGAGGAAGTTGT GGGTTTTTTGGCTTTCGTTTCTGGGCGTAGGTTCGC GTGCGGTTTTCTGGGTGTTTTTTGTGGACTTTAACC GTTACGTCATTTTTTAGTCCTATATATACTCGCTCTGT ACTTGGCCCTTTTTACACTGTGACTGATTGAGCTGG TGCCGTGTCGAGTGGTGTTTTTTAATAGGTTTTTTTA CTGGTAAGGCTGACTGTTATGGCTGCCGCTGTGGAA GCGCTGTATGTTGTTCTGGAGCGGGAGGGTGCTATT TTGCCTAGGCAGGAGGGTTTTTCAGGTGTTTATGTG TTTTTCTCTCCTATTAATTTTGTTATACCTCCTATGGG GGCTGTAATGTTGTCTCTACGCCTGCGGGTATGTATT CCCCCGGGCTATTTCGGTCGCTTTTTAGCACTGACC GATGTTAACCAACCTGATGTGTTTACCGAGTCTTAC ATTATGACTCCGGACATGACCGAGGAACTGTCGGTG GTGCTTTTTAATCACGGTGACCAGTTTTTTTACGGT CACGCCGGCATGGCCGTAGTCCGTCTTATGCTTATA AGGGTTGTTTTTCCTGTTGTAAGACAGGCTTCTAAT GTTTAAATGTTTTTTTTTTTGTTATTTTATTTTGTGTT TAATGCAGGAACCCGCAGACATGTTTGAGAGAAAA ATGGTGTCTTTTTCTGTGGTGGTTCCGGAACTTACC TGCCTTTATCTGCATGAGCATGACTACGATGTGCTTG CTTTTTTGCGCGAGGCTTTGCCTGATTTTTTGAGCA GCACCTTGCATTTTATATCGCCGCCCATGCAACAAG CTTACATAGGGGCTACGCTGGTTAGCATAGCTCCGA GTATGCGTGTCATAATCAGTGTGGGTTCTTTTGTCAT GGTTCCTGGCGGGGAAGTGGCCGCGCTGGTCCGTG CAGACCTGCACGATTATGTTCAGCTGGCCCTGCGAA GGGACCTACGGGATCGCGGTATTTTTGTTAATGTTC CGCTTTTGAATCTTATACAGGTCTGTGAGGAACCTG AATTTTTGCAATCATGATTCGCTGCTTGAGGCTGAA GGTGGAGGGCGCTCTGGAGCAGATTTTTACAATGG CCGGACTTAATATTCGGGATTTGCTTAGAGACATATT GATAAGGTGGCGAGATGAAAATTATTTGGGCATGGT TGAAGGTGCTGGAATGTTTATAGAGGAGATTCACCC |
| | | TGAAGGGTTTAGCCTTTACGTCCACTTGGACGTGAG GGCAGTTTGCCTTTTGGAAGCCATTGTGCAACATCT TACAAATGCCATTATCTGTTCTTTGGCTGTAGAGTTT GACCACGCCACCGGAGGGGAGCGCGTTCACTTAAT AGATCTTCATTTTGAGGTTTTGGATAATCTTTTGGAA TAAAAAAAAAAAAACATGGTTCTTCCAGCTCTTCC CGCTCCTCCCGTGTGTGACTCGCAGAACGAATGTGT AGGTTGGCTGGGTGTGGCTTATTCTGCGGTGGTGGA TGTTATCAGGGCAGCGGCGCATGAAGGAGTTTACAT AGAACCCGAAGCCAGGGGGCGCCTGGATGCTTTGA GAGAGTGGATATACTACAACTACTACACAGAGCGA GCTAAGCGACGAGACCGGAGACGCAGATCTGTTTG TCACGCCCGCACCTGGTTTTGCTTCAGGAAATATGA CTACGTCCGGCGTTCCATTTGGCATGACACTACGAC CAACACGATCTCGGTTGTCTCGGCGCACTCCGTACA GTAGGGATCGCCTACCTCCTTTTGAGACAGAGACCC GCGCTACCATACTGGAGGATCATCCGCTGCTGCCCG AATGTAACACTTTGACAATGCACAACGTGAGTTACG TGCGAGGTCTTCCCTGCAGTGTGGGATTTACGCTGA TTCAGGAATGGGTTGTTCCCTGGGATATGGTTCTGA CGCGGGAGGAGCTTGTAATCCTGAGGAAGTGTATG CACGTGTGCCTGTGTTGTGCCAACATTGATATCATG ACGAGCATGATGATCCATGGTTACGAGTCCTGGGCT CTCCACTGTCATTGTTCCAGTCCCGGTTCCCTGCAG TGCATAGCCGGCGGGCAGGTTTTGGCCAGCTGGTTT AGGATGGTGGTGGATGGCGCCATGTTTAATCAGAGG TTTATATGGTACCGGGAGGTGGTGAATTACAACATG CCAAAAGAGGTAATGTTTATGTCCAGCGTGTTTATG AGGGGTCGCCACTTAATCTACCTGCGCTTGTGGTAT GATGGCCACGTGGGTTCTGTGGTCCCCGCCATGAG CTTTGGATACAGCGCCTTGCACTGTGGGATTTTGAA CAATATTGTGGTGCTGTGCTGCAGTTACTGTGCTGA TTTAAGTGAGATCAGGGTGCGCTGCTGTGCCCGGA GGACAAGGCGTCTCATGCTGCGGGCGGTGCGAATC ATCGCTGAGGAGACCACTGCCATGTTGTATTCCTGC |
| | | AGGACGGAGCGGCGGCGGCAGCAGTTTATTCGCGC GCTGCTGCAGCACCACCGCCCTATCCTGATGCACGA TTATGACTCTACCCCCATGTAGGCGTGGACTTCCCC TTCGCCGCCCGTTGAGCAACCGCAAGTTGGACAGC AGCCTGTGGCTCAGCAGCTGGACAGCGACATGAAC TTAAGCGAGCTGCCCGGGGAGTTTATTAATATCACT GATGAGCGTTTGGCTCGACAGGAAACCGTGTGGAA TATAACACCTAAGAATATGTCTGTTACCCATGATATG ATGCTTTTTAAGGCCAGCCGGGGAGAAAGGACTGT GTACTCTGTGTGTTGGGAGGGAGGTGGCAGGTTGA ATACTAGGGTTCTGTGAGTTTGATTAAGGTACGGTG ATCAATATAAGCTATGTGGTGGTGGGGCTATACTACT GAATGAAAAATGACTTGAAATTTTCTGCAATTGAAA AATAAACACGTTGAAACATAACATGCAACAGGTTC ACGATTCTTTATTCCTGGGCAATGTAGGAGAAGGTG TAAGAGTTGGTAGCAAAAGTTTCAGTGGTGTATTTT CCACTTTCCCAGGACCATGTAAAAGACATAGAGTA AGTGCTTACCTCGCTAGTTTCTGTGGATTCACTAGA ATCGATGTAGGATGTTGCCCCTCCTGACGCGGTAGG AGAAGGGGAGGGTGCCCTGCATGTCTGCCGCTGCT CTTGCTCTTGCCGCTGCTGAGGAGGGGGGCGCATC TGCCGCAGCACCGGATGCATCTGGGAAAAGCAAAA AAGGGGCTCGTCCCTGTTTCCGGAGGAATTTGCAA GCGGGGTCTTGCATGACGGGGAGGCAAACCCCCGT TCGCCGCAGTCCGGCCGGCCCGAGACTCGAACCGG GGGTCCTGCGACTCAACCCTTGGAAAATAACCCTC CGGCTACAGGGAGCGAGCCACTTAATGCTTTCGCTT TCCAGCCTAACCGCTTACGCCGCGCGCGGCCAGTG GCCAAAAAAGCTAGCGCAGCAGCCGCCGCGCCTG GAAGGAAGCCAAAAGGAGCGCTCCCCCGTTGTCTG ACGTCGCACACCTGGGTTCGACACGCGGGCGGTAA CCGCATGGATCACGGCGGACGGCCGGATCCGGGGT TCGAACCCCGGTCGTCCGCCATGATACCCTTGCGAA TTTATCCACCAGACCACGGAAGAGTGCCCGCTTAC AGGCTCTCCTTTTGCACGGTCTAGAGCGTCAACGA |
| | | CTGCGCACGCCTCACCGGCCAGAGCGTCCCGACCA TGGAGCACTTTTTGCCGCTGCGCAACATCTGGAAC CGCGTCCGCGACTTTCCGCGCGCCTCCACCACCGC CGCCGGCATCACCTGGATGTCCAGGTACATCTACGG ATTACGTCGACGTTTAAACCATATGATCAGCTCACT CAAAGGCGGTAATACGGTTATCCACAGAATCAGGG GATAACGCAGGAAAGAACATGTGAGCAAAAGGCC AGCAAAAGGCCAGGAACCGTAAAAAGGCCGCGTT GCTGGCGTTTTTCCATAGGCTCCGCCCCCCTGACGA GCATCACAAAAATCGACGCTCAAGTCAGAGGTGGC GAAACCCGACAGGACTATAAAGATACCAGGCGTTT CCCCCTGGAAGCTCCCTCGTGCGCTCTCCTGTTCCG ACCCTGCCGCTTACCGGATACCTGTCCGCCTTTCTC CCTTCGGGAAGCGTGGCGCTTTCTCATAGCTCACGC TGTAGGTATCTCAGTTCGGTGTAGGTCGTTCGCTCC AAGCTGGGCTGTGTGCACGAACCCCCCGTTCAGCC CGACCGCTGCGCCTTATCCGGTAACTATCGTCTTGA GTCCAACCCGGTAAGACACGACTTATCGCCACTGG CAGCAGCCACTGGTAACAGGATTAGCAGAGCGAGG TATGTAGGCGGTGCTACAGAGTTCTTGAAGTGGTGG CCTAACTACGGCTACACTAGAAGAACAGTATTTGGT ATCTGCGCTCTGCTGAAGCCAGTTACCTTCGGAAAA AGAGTTGGTAGCTCTTGATCCGGCAAACAAACCAC CGCTGGTAGCGGTGGTTTTTTTGTTTGCAAGCAGCA GATTACGCGCAGAAAAAAAGGATCTCAAGAAGATC CTTTGATCTTTTCTACGGGGTCTGACGCTCAGTGGA ACGAAAACTCACGTTAAGGGATTTTGGTCATGAGAT TATCAAAAAGGATCTTCACCTAGATCCTTTTAAATTA AAAATGAAGTTTTAAATCAATCTAAAGTATATATGAG TAAACTTGGTCTGACAGTTACCAATGCTTAATCAGT GAGGCACCTATCTCAGCGATCTGTCTATTTCGTTCAT CCATAGTTGCCTGACTCCCCGTCGTGTAGATAACTA CGATACGGGAGGGCTTACCATCTGGCCCCAGTGCTG CAATGATACCGCGAGACCCACGCTCACCGGCTCCA GATTTATCAGCAATAAACCAGCCAGCCGGAAGGGC |
| | | CGAGCGCAGAAGTGGTCCTGCAACTTTATCCGCCT CCATCCAGTCTATTAATTGTTGCCGGGAAGCTAGAG TAAGTAGTTCGCCAGTTAATAGTTTGCGCAACGTTG TTGCCATTGCTACAGGCATCGTGGTGTCACGCTCGT CGTTTGGTATGGCTTCATTCAGCTCCGGTTCCCAAC GATCAAGGCGAGTTACATGATCCCCCATGTTGTGCA AAAAAGCGGTTAGCTCCTTCGGTCCTCCGATCGTTG TCAGAAGTAAGTTGGCCGCAGTGTTATCACTCATGG TTATGGCAGCACTGCATAATTCTCTTACTGTCATGCC ATCCGTAAGATGCTTTTCTGTGACTGGTGAGTACTC AACCAAGTCATTCTGAGAATAGTGTATGCGGCGACC GAGTTGCTCTTGCCCGGCGTCAATACGGGATAATAC CGCGCCACATAGCAGAACTTTAAAAGTGCTCATCAT TGGAAAACGTTCTTCGGGGCGAAAACTCTCAAGGA TCTTACCGCTGTTGAGATCCAGTTCGATGTAACCCA CTCGTGCACCCAACTGATCTTCAGCATCTTTTACTTT CACCAGCGTTTCTGGGTGAGCAAAAACAGGAAGG CAAAATGCCGCAAAAAAGGGAATAAGGGCGACAC GGAAATGTTGAATACTCATACTCTTCCTTTTTCAATA TTATTGAAGCATTTATCAGGGTTATTGTCTCATGAGC GGATACATATTTGAATGTATTTAGAAAAATAAACAAA TAGGGGTTCCGCGCACATTTCCCCGAAAAGTGCCA CCTAAATTGTAAGCGTTAATATTTTGTTAAAATTCGC GTTAAATTTTTGTTAAATCAGCTCATTTTTTAACCAA TAGGCCGAAATCGGCAAAATCCCTTATAAATCAAAA GAATAGACCGAGATAGGGTTGAGTGTTGTTCCAGTT TGGAACAAGAGTCCACTATTAAAGAACGTGGACTC CAACGTCAAAGGGCGAAAAACCGTCTATCAGGGCG ATGGCCCACTACGTGAACCATCACCCTAATCAAGTT TTTTGGGGTCGAGGTGCCGTAAAGCACTAAATCGG AACCCTAAAGGGAGCCCCCGATTTAGAGCTTGACG GGGAAAGCCGGCGAACGTGGCGAGAAAGGAAGGG AAGAAAGCGAAAGGAGCGGGCGCTAGGGCGCTGG CAAGTGTAGCGGTCACGCTGCGCGTAACCACCACA CCCGCCGCGCTTAATGCGCCGCTACAGGGCGCGAT |
| | | GGATCC |

All documents mentioned in the present disclosure are incorporated herein by reference as if each document were individually incorporated by reference. In addition, it should be understood that after reading the above teachings of the present disclosure, those skilled in the art may make various changes or modifications to the present disclosure, and such equivalent forms shall also fall within the scope defined by the appended claims of the present disclosure.

## Claims

1. An expression cassette, comprising the following elements: a promoter, optionally a nucleotide sequence encoding a first signal peptide, a nucleotide sequence encoding a heavy chain of an anti-IgE antibody, optionally a nucleotide sequence encoding a linker, optionally a nucleotide sequence encoding a second signal peptide, and a nucleotide sequence encoding a light chain of an anti-IgE antibody.

2. The expression cassette according to claim 1, wherein the linker comprises one or more selected from the group consisting of a self-cleaving linker, a Furin cleavage site, and an IRES linker.

3. The expression cassette according to claim 1, wherein the anti-IgE antibody is selected from the group consisting of Ligelizumab, Omalizumab, or a combination thereof.

4. The expression cassette according to claim 1, wherein the expression cassette comprises a structure of Formula I from the 5' end to the 3' end:
Z1-Z2-Z3-Z4-Z5-Z6-Z7-Z8-Z9 (Formula I)
wherein each "-" is independently selected from a bond or a nucleotide linking sequence;
Z1 is a promoter;
Z2 optionally is a nucleotide sequence encoding the first signal peptide;
Z3 is a nucleotide sequence encoding the heavy chain of an anti-IgE antibody;
Z4 is a Furin cleavage site;
Z5 is a nucleotide sequence encoding the self-cleaving linker;
Z6 optionally is a nucleotide sequence encoding a second signal peptide; and
Z7 is a nucleotide sequence encoding the light chain of the anti-IgE antibody;
Z8 optionally is a woodchuck hepatitis virus posttranscriptional regulatory element (WPRE element);
Z9 optionally is a polyadenylation signal (polyA).

5. The expression cassette according to claim 1, wherein the expression cassette comprises a structure of Formula II from the 5' end to the 3' end:
*Z*1-*Z*2-*Z*3-*Z*4'-*Z*6-*Z*7-*Z*8-*Z*9 (Formula II)
wherein Z1, Z2, Z3, Z6, Z7, Z8, and Z9 are defined as above, and Z4' is an IRES element;
each "-" is independently selected from a bond or a nucleotide linking sequence.
optionally, the nucleotide sequence of the expression cassette is selected from the group consisting of:
(a) a nucleotide sequence shown in SEQ ID NO:7;
(b) a nucleotide sequence having ≥ 95% (optionally ≥ 98%, optionally ≥ 99%) sequence identity to the nucleotide sequence shown in SEQ ID NO:7; and
(c) a nucleotide sequence complementary to the nucleotide sequence described in (a) or (b).

6. A vector, comprising the expression cassette according to claim 1.

7. The vector according to claim 6, wherein the vector is an adeno-associated virus (AAV) vector.

8. An adeno-associated virus vector, comprising the expression cassette according to claim 1.

9. A host cell, comprising the vector according to claim 6 or the adeno-associated virus vector according to claim 8, or a chromosome thereof carrying the exogenous expression cassette according to claim 1.

10. A use of the vector according to claim 6, the adeno-associated virus vector according to claim 8, or the host cell according to claim 9 in the preparation of a formulation or composition for treating allergic diseases.

11. The use according to claim 10, wherein the allergic diseases are selected from the group consisting of allergic asthma, chronic spontaneous urticaria, or a combination thereof.

12. A pharmaceutical formulation, comprising (a) the vector according to claim 6, the adeno-associated virus vector according to claim 8, or the host cell according to claim 9, and (b) a pharmaceutically acceptable carrier or excipient.

13. A method for preparing a recombinant adeno-associated virus vector, comprising culturing the host cell according to claim 9 to obtain the recombinant adeno-associated virus vector.

14. A kit, comprising:
(i) a first container and an active ingredient (a) in the first container, wherein the active ingredient (a) is selected from the group consisting of the vector according to claim 6, the adeno-associated virus vector according to claim 8, the host cell according to claim 9, or the pharmaceutical formulation according to claim 12;
(ii) optionally a second container and an active ingredient (b) in the second container, wherein the active ingredient (b) is selected from another drug for treating allergic diseases or an active ingredient thereof.
